# EUROPEAN PATENT APPLICATION

(11) **EP 4 516 773 A1**
(43) Date of publication of application: **05.03.2025**
(21) Application number: 24767055.7
(22) Date of filing: 01.03.2024
(51) Int. Cl.: C07C 69/708, C07C 69/712, C07C 229/12, C07C 235/08, C07C 235/10, C07C 235/16, C07C 235/32, C07C 271/14, C07C 275/34, C09K 3/18, D06M 13/236, D21H 17/34, D21H 19/20, D21H 21/16

(54) **FLUORINE-CONTAINING COMPOUND**

(30) Priority: 03.03.2023 JP 2023033137
(71) Applicant: DAIKIN INDUSTRIES, LTD., Osaka-shi, Osaka 530-0001 (JP)
(72) Inventor: HIGASHI, Masahiro, Osaka-Shi, Osaka 530-0001 (JP); AKUTA, Ryo, Osaka-Shi, Osaka 530-0001 (JP); SAKAMAKI, Tatsunori, Osaka-Shi, Osaka 530-0001 (JP); SHIBATA, Shun, Osaka-Shi, Osaka 530-0001 (JP); YAMAMOTO, Ikuo, Osaka-Shi, Osaka 530-0001 (JP); KASHIWAGI, Masato, Osaka-Shi, Osaka 530-0001 (JP); MATSUDA, Michio, Osaka-Shi, Osaka 530-0001 (JP); FUKUMORI, Masaki, Osaka-Shi, Osaka 530-0001 (JP)
(74) Representative: Hoffmann Eitle
(86) International application number: PCT/JP2024/007769
(87) International publication number: WO 2024/185687

(57) **Abstract**

The present invention provides a novel fluorine-containing compound which has a moiety derived from a compound (a) that has one or more active hydrogen-containing groups selected from among a hydroxy group, an amino group, a carboxyl group and a thiol group, and a moiety derived from a compound (b) that has R^{f1} or R^{f2}, wherein: R^{f1} is -CF₃, -CF₂H or -CFH₂; R^{f2} is -CF₂- or - CFH-; and R^{f1} and R^{f2} are not parts of a fluoroalkyl group having 2 or more carbon atoms.

## Description

### Technical Field

The present disclosure relates to a fluorine-containing compound.

### Background Art

Certain non-fluorine polymers are known to impart oil resistance when used for surface treatment of substrates (Patent Literature 1).

### Citation List

### Patent Literature

[Patent Literature 1] WO2020-054856

### Summary of Invention

### Technical Problem

An object of the present disclosure is to provide a novel fluorine-containing compound.

### Solution to Problem

The present disclosure includes the following embodiments.

### [Item 1]

A fluorine-containing compound comprising:
a moiety derived from a compound (a) having one or more active hydrogen-containing groups selected from a hydroxy group, an amino group, a carboxyl group and a thiol group; and a moiety derived from a compound (b) having R^{f1} or R^{f2}, wherein
R^{f1} is -CF₃, -CF₂H or -CFH₂,
R^{f2} is -CF₂- or -CFH-, and
R^{f1} and R^{f2} do not constitute a moiety of a fluoroalkyl group having 2 or more carbon atoms.

### [Item 2]

The fluorine-containing compound according to item 1, wherein the fluorine-containing compound has an oxygen atom or a nitrogen atom in a position adjacent to the R^{f1}, and has an oxygen atom or a nitrogen atom in at least one of positions adjacent to the R^{f2}.

### [Item 3]

The fluorine-containing compound according to item 1 or 2, wherein
the R^{f1} is CF₃-, and
the R^{f2} is -CF₂-.

### [Item 4]

The fluorine-containing compound according to any one of items 1 to 3, wherein the compound (b) has a group represented by the formula:

-Y^{f-}Z^{f}_{α}

wherein, independently at each occurrence,
Y^{f} is a 1+α valent group composed of one or more selected from the group consisting of Y^{f1} and y^{f2},
Y^{f1} is a group composed of one or more selected from a direct bond, -O-, -C(=O)-, -C(=NR')-, -S-, -S(=O)₂-, - NR'-, -C(OR')R'-, -C(OR')(-)₂ and -N(-)₂, wherein R' is a hydrogen atom or an organic group,
Y^{f2} is a group composed of one or more selected from the group consisting of an aliphatic group and an aromatic group,
Z^{f} is R^{f1}, or a hydrocarbon group having 2 or more and 40 or less carbon atoms and including R^{f1} or R^{f2}, and
α is 1 to 3.

### [Item 5]

The fluorine-containing compound according to any one of items 1 to 4, wherein the compound (b) has a group represented by the formula:

-Y^{f11}- (Y^{f21}-_{Y}^{f12}) _{β-}Y^{f22-}Y^{f13}-Z^{f}

wherein, independently at each occurrence,
Y^{f11} is a direct bond, -O-, -NH-, -C(=O)-, -C(=O)-NH- or -S-,
Y^{f21} is a group composed of one or more selected from the group consisting of an aliphatic group and an aromatic group,
Y^{f12} is a group composed of one or more selected from -O-, -C(=O)-, -C(=NR')-, -S-, -S(=O)₂-, -NR'- and - C(OR')R'-, wherein R' is a hydrogen atom or an organic group,
β is 0 to 3,
Y^{f22} is a group composed of one or more selected from the group consisting of an aliphatic group and an aromatic group,
Y^{f13} is -O-, -O-C(=O)-O-, -O-C(=O)-NR'-, -NR'-, -NR'-C(=O)-O-, -NR'-C(=O)-NR'-, -C(=O)-O-, -C(=O)-NR'- or - SO₂NR'-, wherein R' is a hydrogen atom or an organic group, and
Z^{f} is R^{f1}, or a hydrocarbon group having 2 or more and 40 or less carbon atoms and including R^{f1} or R^{f2}.

### [Item 6]

The fluorine-containing compound according to item 5, wherein
Y^{f22} is a group represented by the formula: -Y^{f221}-Y^{f222}-
wherein
y^{f221} is a direct bond or a hydrocarbon group having 2 to 40 carbon atoms, and
Y^{f222} is a direct bond or a phenyl group,
Y^{f13} is -O- or -NR'-, wherein R' is a hydrogen atom or an organic group,
β is 0 or 1, and
Z^{f} is R^{f1}.

### [Item 7]

The fluorine-containing compound according to any one of items 1 to 6, wherein the compound (b) is an active hydrogen-reactive compound; a polymerizable monomer or a polymer thereof.

### [Item 8]

The fluorine-containing compound according to any one of items 1 to 7, wherein the compound (a) is a natural product.

### [Item 9]

The fluorine-containing compound according to any one of items 1 to 8, wherein the compound (a) is polyol, polyamine or polycarboxylic acid.

### [Item 10]

The fluorine-containing compound according to any one of items 1 to 9, wherein the compound (a) is a monosaccharide, an oligosaccharide, a polysaccharide, a sugar alcohol, a hydroxy acid, an amino acid, vitamin, flavonol, hydroxyhydrocarbon, a polymer of a hydroxy group-containing compound, an organic acid, an organic amine, or a saturated/unsaturated aliphatic hydrocarbon compound.

### [Item 11]

The fluorine-containing compound according to any one of items 1 to 10, wherein the compound (a) is
glucose, fructose, galactose, xylose;
sucrose, cycloamylose, cyclodextrin, maltose, trehalose, lactose, sucralose;
sorbitol, maltitol, erythritol, isomalt, lactitol, mannitol, xylitol, sorbitan, lactitol;
starch, cellulose, curdlan, pullulan, alginic acid, carrageenan, guar gum, chitin, chitosan, locust bean gum, kappa-carrageenan, iota-carrageenan, isomaltodextrin, gellan gum, tamarind seed gum;
ascorbic acid, kojic acid, quinic acid, chlorogenic acid, gluconic acid;
glucosamine;
inositol;
catechin, quercetin, anthocyanin;
glycerol, ethylene glycol, propylene glycol, diethylene glycol, triethylene glycol, tetraethylene glycol, neopentyl glycol, trimethylene glycol, trimethylolpropane, trimethylolethane;
polyglycerol, polyvinyl alcohol, hydroxyethyl (meth)acrylate polymer, hydroxypropyl (meth)acrylate polymer and hydroxybutyl(meth)acrylate polymer;
citric acid, malic acid, glutaric acid, adipic acid, phthalic acid, alginic acid, tartaric acid, oxalic acid, malonic acid, succinic acid, fumaric acid, maleic acid, aldaric acid;
tricarballylic acid, t-aconitic acid, trimellitic acid;
pyromellitic acid;
alkylene diamine, alkylene triamine, aromatic diamine; or
a saturated/unsaturated aliphatic hydrocarbon compound having an active hydrogen group.

### [Item 12]

The fluorine-containing compound according to any one of items 1 to 11, wherein the fluorine-containing compound has a biobased content of 20% or more.

### [Item 13]

The fluorine-containing compound according to any one of items 1 to 12, wherein the fluorine-containing compound has a weight average molecular weight of 1,000 or more.

### [Item 14]

The fluorine-containing compound according to any one of items 1 to 12, wherein the fluorine-containing compound has a weight average molecular weight of less than 1,000.

### [Item 15]

The fluorine-containing compound according to any one of items 1 to 14, wherein
the fluorine-containing compound has a monovalent hydrocarbon group having 6 or more and 40 or less carbon atoms as a modifying group other than -Y^{f}-Z^{f}ₙ.

### [Item 16]

The fluorine-containing compound according to item 11, wherein the compound (b) has a group represented by the formula:

-Y^{f11}-(Y^{f21}-Y^{f12})_{β}-Y^{f22-}Y^{f13}-Z^{f}

wherein, independently at each occurrence,
Y^{f11} is a direct bond, -O-, -NH-, -C(=O)-, -C(=O)-NH- or -S-,
Y^{f21} is a group composed of one or more selected from the group consisting of an aliphatic group and an aromatic group,
Y^{f12} is a group composed of one or more selected from -O-, -C(=O)-, -C(=NR')-, -S-, -S(=O)₂-, -NR'- and - C(OR')R'-, wherein R' is a hydrogen atom or an organic group,
β is 0 to 3,
Y^{f22} is a group composed of one or more selected from the group consisting of an aliphatic group and an aromatic group,
Y^{f13} is -O-, -O-C(=O)-O-, -O-C(=O)-NR'-, -NR'-, -NR'-C(=O)-O-, -NR'-C(=O)-NR'-, -C(=O)-O-, -C(=O)-NR'- or - SO₂NR'-, wherein R' is a hydrogen atom or an organic group, and
Z^{f} is -CF₃.

### [Item 17]

A dispersion comprising the fluorine-containing compound according to any one of items 1 to 16 and a liquid medium.

### [Item 18]

A water- and oil-repellent agent comprising the fluorine-containing compound according to any one of items 1 to 16.

### [Item 19]

The water- and oil-repellent agent according to item 18, which is for a textile product or a paper product.

### [Item 20]

A method for producing a treated substrate, comprising treating a substrate with the water- and oil-repellent agent according to item 18 or 19.

### [Item 21]

The method according to item 20, wherein the substrate is a textile product or a paper product.

### [Item 22]

A product to which the fluorine-containing compound according to any one of items 1 to 16 is adhered.

### Advantageous Effect of Invention

The present disclosure can provide a novel fluorine-containing compound.

### Description of Embodiments

### <Definition of Terms>

As used herein, the "n valent group" refers to a group having n bonds, i.e., a group forming n bonds. The "n valent organic group" refers to a n valent group containing carbon. Such organic groups are not limited, but can be hydrocarbon groups or derivatives thereof. The derivative of the hydrocarbon group refers to a group that has one or more of N, O, S, Si, amide, sulfonyl, siloxane, carbonyl, carbonyloxy, halogen and the like at the end or in the molecular chain of a hydrocarbon group.

As used herein, the "hydrocarbon group" refers to a group containing carbon and hydrogen and a group in which a hydrogen atom is removed from the hydrocarbon. Such hydrocarbon groups are not limited, but include C₁₋₄₀ hydrocarbon groups, such as an aliphatic hydrocarbon group and an aromatic hydrocarbon group. The above "aliphatic hydrocarbon group" may be either linear, branched, or cyclic, and may be either saturated or unsaturated. The hydrocarbon group may include one or more ring structures. In an explicit term, the hydrocarbon group may be substituted by one or more substituents.

Whether or not the phrases "independently at each occurrence", "independently with each other", "each independently" or similar expressions are explicitly described herein, unless otherwise described that they are exceptions, when a plurality of terms (symbols) that can occur in a chemical structure is defined, such definition is applied independently to each occurrence.

The chemical structures described herein should be understood not to encompass chemical structures that are recognized by those skilled in the art as being chemically impossible or extremely unstable.

### <Fluorine-containing compound>

The fluorine-containing compound in the present disclosure comprises:
(a) a moiety derived from a compound having one or more groups selected from a hydroxy group, an amino group, a carboxyl group and a thiol group; and
(b) a moiety derived from a compound having R^{f1} or R^{f2}.

The fluorine-containing compound may be formed by the reaction between active hydrogen that the compound (a) has and the active hydrogen-reactive group that the compound (b) has. The fluorine-containing compound may also be formed by graft polymerization at the active site in the molecular chain of the compound (a) with the compound (b).

### {Characteristics of fluorine-containing compound}

The fluorine-containing compound in the present disclosure adheres to a substrate and imparts liquid repellency (e.g., oil-repellency, water-repellency, oil resistance, water resistance) to the substrate.

The fluorine-containing compound may have a HD (n-hexadecane) contact angle of 10° or more, 15° or more, 25° or more, 35° or more, 40° or more, 45° or more, 55° or more, or 65° or more, and preferably 20° or more, and particularly 30° or more, and 100° or less, 90° or less or 75° or less. A HD contact angle of the fluorine-containing compound of the lower limit or more can impart good liquid-repellency (in particular oil-repellency) to a substrate. The HD contact angle is a static contact angle of a fluorine-containing compound to a spin-coated film, which is obtained by dropping 2 µL of HD on a spin-coated film and measuring the contact angle one second after the droplet reaches the film.

The fluorine-containing compound may have a water contact angle of 20° or more, 25° or more, 30° or more, 35° or more, 40° or more, 45° or more, 50° or more, 55° or more, 65° or more, 75° or more, 85° or more, 90° or more, or 100° or more, and 160° or less, 140° or less, 130° or less, 120° or less, 110° or less, 100° or less or 90° or less. A water contact angle of the fluorine-containing compound of the lower limit or more can impart good liquid-repellency (in particular water-repellency) to a substrate. The water contact angle is a static contact angle of a fluorine-containing compound to a spin-coated film, which is obtained by dropping 2 µL of water on a spin-coated film and measuring the contact angle one second after the droplet reaches the film.

The fluorine-containing compound may have a melting point or a glass transition temperature (e.g., a melting point) of 0°C or higher, 20°C or higher, 30°C or higher, 35°C or higher, 40°C or higher, 45°C or higher, 50°C or higher, or 55°C or higher, and 200°C or lower, 150°C or lower, 100°C or lower, 80°C or lower, or 70°C or lower. The fluorine-containing compound has a melting point of usually 30°C or higher, preferably 40°C or higher, and for example, 75°C or higher.

The fluorine-containing compound is preferably a compound with carbon of biobased origin. A biobased content is measured in accordance with ASTM D6866. The biobased content of the fluorine-containing compound may be 20% or more, preferably 30% or more, more preferably 50% or more, further preferably 60% or more, further preferably 70% or more, and most preferably 80% or more or 90% or more, and for example, it is 100%. The high biobased content refers to less amount of fossil resource-based material used, which is represented by petroleum or the like, and from this perspective, it can be said that the higher the biobased content of the fluorine-containing compound, the more preferable it is**.**

The fluorine-containing compound in the present disclosure comprises R^{f1} or R^{f2} described above. Meanwhile, the fluorine-containing compound in the present disclosure may not have any one selected from the group consisting of a fluoroalkyl group having 8 or more carbon atoms, a perfluoroalkyl group having 8 or more carbon atoms, a fluoroalkyl group having 4 or more carbon atoms, a perfluoroalkyl group having 4 or more carbon atoms, a fluoroalkyl group having 2 or more carbon atoms and a perfluoroalkyl group having 2 or more carbon atoms. The fluorine-containing compound in the present disclosure can impart liquid-repellency without including these fluorine-containing groups to a substrate. As used herein, the fluoroalkyl group may mean an alkyl group in which one or more hydrogen atoms at the respective carbon atoms are substituted by a fluorine atom.

The fluorine-containing compound may be a low molecular weight compound (having a weight average molecular weight of less than 1,500, less than 1,000 or 500 or less) and/or a high molecular weight compound. The fluorine-containing compound may have a weight average molecular weight of 100 or more, 200 or more, 300 or more, 400 or more, 500 or more, 1,000 or more, 3,000 or more, 5,000 or more, 10,000 or more, 30,000 or more, 100,000 or more, 300,000 or more, or 500,000 or more, and may have a weight average molecular weight of 1,000,000 or less, 750,000 or less, 500,000 or less, 3,000,000 or less, 100,000 or less, 75,000 or less, 50,000 or less, 30,000 or less, 10,000 or less, 9,000 or less, 8,000 or less, 7,000 or less, 6,000 or less, 5,000 or less, 3,000 or less, 2,000 or less, 1,000 or less, or 500 or less.

The substitution ratio by active hydrogen in the fluorine-containing compound may be 0.01% or more, 0.1% or more, 1% or more, 3% or more, 5% or more, 10% or more, 20% or more, 30% or more, 40% or more, 50% or more, 60% or more, 70% or more, 80% or more, 90% or more, or 100%, and preferably 10% or more, for example, 30% or more, 40% or more, 50% or more, 60% or more, 70% or more, and particularly 80% or more, and may be 100% or less, 95% or less, 85% or less, 75% or less, 65% or less, 55% or less, 45% or less, 35% or less, 25% or less, 15% or less, and for example, 95% or less. In this regard, the "substitution ratio" means the proportion (mol%) of the modified active hydrogen out of the active hydrogen derived from the compound (a), which may mean the proportion (mol%) of the active hydrogen modified by the structure derived from the compound (b).

The residual ratio of active hydrogen in the fluorine-containing compound may be 1% or more, 3% or more, 5% or more, 10% or more, 20% or more, 30% or more, 40% or more, 50% or more, 60% or more, 70% or more, 80% or more, or 90% or more, and for example, 5% or more, and may be 100% or less, 95% or less, 85% or less, 75% or less, 65% or less, 55% or less, 45% or less, 35% or less, 25% or less, 15% or less, or 5% or less, and for example, 50% or less, 30% or less, or 10% or less. In this regard, the "residual ratio" means the proportion (mol%) of active hydrogen without modification, out of the active hydrogen derived from the compound (a).

The fluorine-containing compound may have 2 or more, 5 or more, 7 or more, 10 or more, 15 or more, 30 or more, or 50 or more modifying groups, and 20,000 or less, 10,000 or less, 5,000 or less, 1,000 or less, 750 or less, 500 or less, 300 or less, 100 or less, 50 or less, 30 or less, or 20 or less modifying groups. The modifying group may be a moiety derived from the compound (b).

The equivalent of the modifying group in the fluorine-containing compound may be 100 or more, 150 or more, 250 or more, 350 or more, 450 or more, 550 or more, 650 or more, 750 or more, or 1,000 or more, and 10,000 or less, 5,000 or less, 2,500 or less, 2,000 or less, 1,500 or less, 1,000 or less, 750 or less, 500 or less, or 400 or less. Dividing the weight average molecular weight of the fluorine-containing compound by the number of modifying groups gives the equivalent of the modifying group. The modifying group may be a moiety derived from the compound (b).

### {R^{f1} and R^{f2}}

The fluorine-containing compound has R^{f1} or R^{f2}, and for example, In this regard, R^{f1} and R^{f2} do not constitute a moiety of a fluoroalkyl group having 2 or more carbon atoms. R^{f2} may not be a moiety of -CH₂-CF₂-CH₂- .

R^{f1} is -CF₃, -CF₂H or -CFH₂, and preferably -CF₃. The fluorine-containing compound may have an oxygen atom or a nitrogen atom in the position adjacent to

R^{f2} is -CF₂- or -CFH-, and preferably -CF₂-. The fluorine-containing compound may have an oxygen atom or a nitrogen atom in at least one of the positions adjacent to R^{f2}.

### [Compound (a)]

The compound (a) has one or more active hydrogen-containing groups selected from a hydroxy group, an amino group, a carboxyl group and a thiol group, and is a raw material of the fluorine-containing compound. The compound (a) may be aliphatic or aromatic, and is preferably aliphatic. Most of the compounds having one or more active hydrogen-containing groups selected from a hydroxy group, an amino group, a carboxyl group and a thiol group are derived from a natural product, and this allows the biobased content of the fluorine-containing compound to be increased.

It is preferable that the compound (a) has carbon derived from biomass. The biobased content is measured according to ASTM D6866. The fluorine-containing compound may have a biobased content of 20% or more, preferably 30% or more, more preferably 50% or more, even more preferably 60% or more, still more preferably 70% or more, and most preferably 80% or more or 90% or more, and for example, 100%. A high biobased content means that the amount of use of fossil resource materials, which are typically petroleum, is small, and a higher biobased content of the compound (a) is preferred from that point of view.

The compound (a) may be a low molecular weight compound (having a weight average molecular weight of, for example, less than 1,000 or 500 or less) and/or a high molecular weight compound. The compound (a) may have a weight average molecular weight of 100 or more, 300 or more, 500 or more, 1,000 or more, 3,000 or more, 5,000 or more, 10,000 or more, 30,000 or more, 100,000 or more, 300,000 or more, or 500,000 or more, and may have a weight average molecular weight of 5,000,000 or less, 3,000,000 or less, 1,000,000 or less, 750,000 or less, 500,000 or less, 3,000,000 or less, 100,000 or less, 75,000 or less, 50,000 or less, 30,000 or less, 10,000 or less, 5,000 or less, 3,000 or less, 2,000 or less, 1,000 or less, or 500 or less.

The compound (a) may have 2 or more, 5 or more, 7 or more, 10 or more, 15 or more, 30 or more, 50 or more, or 100 or more active hydrogen-containing groups, and may have 20,000 or less, 10,000 or less, 5,000 or less, 3,000 or less, 1,000 or less, 750 or less, 500 or less, 300 or less, 100 or less, 50 or less, 30 or less, or 20 or less active hydrogen-containing groups.

The equivalent of the active hydrogen-containing group in the compound (a) may be 20 or more, 40 or more, 60 or more, 80 or more, 100 or more, 120 or more, or 150 or more, and may be 20,000 or less, 10,000 or less, 5,000 or less, 1,000 or less, 800 or less, 600 or less, 400 or less, 200 or less, 100 or less, or 75 or less. The equivalent of the active hydrogen-containing group of the compound (a) is obtained by dividing the weight average molecular weight of the compound (a) by the number of active hydrogen-containing groups.

The compound (a) may be a natural product. The compound (a) may be a high molecular weight natural product, a low molecular weight natural product, or a derivative thereof. The above natural products also include a compound converted from microorganisms.

The compound (a) may be a compound which has an active hydrogen-containing group selected from a hydroxy group, an amino group, a carboxyl group and a thiol group, and is preferably polyol, polyamine, polycarboxylic acid and polythiol, which have a plurality of active hydrogen-containing groups. The compound (a) may be a compound other than polythiol.

Examples of compounds (a) include a monosaccharide, an oligosaccharide, a polysaccharide, a sugar alcohol (reducing sugar), a hydroxy acid, an amino acid, vitamin, flavonol, polyhydric alcohol, a polymer of a hydroxy group-containing compound, polycarboxylic acid, polyamine and polywax.

Examples of monosaccharides include glucose, fructose, galactose and xylose.

Examples of oligosaccharides include sucrose, cycloamylose, cyclodextrin, maltose, trehalose, lactose and sucralose.

Examples of sugar alcohols (reducing sugar) include sorbitol, maltitol, erythritol, glycerol, isomalt, lactitol, mannitol, xylitol, sorbitan and lactitol.

Examples of polysaccharides include starch, cellulose, curdlan, pullulan, alginic acid, carrageenan, guar gum, chitin, chitosan, locust bean gum, kappa-carrageenan, iota-carrageenan, isomaltodextrin, gellan gum and tamarind seed gum.

Examples of hydroxy acids include kojic acid, quinic acid, chlorogenic acid and gluconic acid.

Examples of amino acids include glucosamine.

Examples of vitamins include ascorbic acid and inositol.

Examples of flavonols include catechin, quercetin and anthocyanin.

Examples of hydroxyhydrocarbons include ethylene glycol, propylene glycol, diethylene glycol, triethylene glycol, tetraethylene glycol, neopentyl glycol, trimethylene glycol, glycerol, trimethylolpropane and trimethylolethane. The hydroxyhydrocarbon is a hydrocarbon having a hydroxy group, and may be aliphatic or aromatic, and is preferably aliphatic. The term "hydroxyhydrocarbon" may also mean a hydroxyhydrocarbon other than the compounds included in a different group, such as polysaccharide (other hydroxyhydrocarbons).

Examples of polymers of a hydroxy group-containing compound include polyglycerol, polyvinyl alcohol, hydroxyethyl (meth)acrylate polymer, hydroxypropyl (meth)acrylate polymer and hydroxybutyl (meth)acrylate polymer.

Examples of polycarboxylic acids include citric acid, malic acid, glutaric acid, adipic acid, phthalic acid, alginic acid, tartaric acid, oxalic acid, malonic acid, succinic acid, fumaric acid, maleic acid, aldaric acid; tricarballylic acid, t-aconitic acid, trimellitic acid; pyromellitic acid and itaconic acid. Polycarboxylic acid may also be a polymer of a polymerizable compound such as (meth)acrylic acid.

Examples of polyamines include alkylene diamine, alkylene triamine and aromatic diamine. Specific examples thereof include ethylenediamine, propylenediamine, butanediamine, pentanediamine, hexamethylenediamine, heptanediamine, octanediamine, 1,3-bis(aminomethylcyclohexane, xylylenediamine, diethyltoluenediamine, 4,4'-diaminodiphenylmethane, dimethylthiotoluenediamine, 4,4'-methylenebis[N-(1-methylpropyl)aniline], aminobenzylamine, bis[2-(4-aminophenyl)-2-propylbenzene, 4,4'-diaminodiphenylether, 3,3'-diaminodiphenylsulfon, 4,4'-diaminodiphenylsulfon, 4,4'-methylenebis[N-(1-methylpropyl)aniline], trimethylene-bis(4-aminobenzoate), diethylenetriamine, 2,2'-diamino-N-methyldiethylamine, triethylenetetramine and tris (2-aminoethyl) amine. Polyamine may also be a polymer of a polymerizable compound such as allylamine.

Examples of saturated/unsaturated aliphatic hydrocarbon compounds having an active hydrogen group include an aliphatic alcohol, an amide group-containing aliphatic alcohol, aliphatic carboxylic acid, an aliphatic hydroxy acid and an aliphatic thiol.

### [Compound (b)]

The compound (b) has R^{f1} or R^{f2}. The compound (b) may have an active hydrogen-reactive group which can react with the active hydrogen or the active hydrogen-containing group that the compound (a) has. The compound (b) may also have a polymerizable (graft-polymerizable) group, for example, an ethylenically unsaturated polymerizable group.

The compound (b) may have a group represented by the formula: -Y^{f}-Z^{f}_{α}
[wherein, independently at each occurrence,
Y^{f} is a 1+α valent group composed of one or more selected from the group consisting of Y^{f1} and Y^{f2},
Y^{f1} is a group composed of one or more selected from a direct bond, -O-, -C(=O)-, -C(=NR')-, -S-, -S(=O)₂-, - NR'-, -C(OR')R'-, -C(OR') (-)₂ and -N(-)₂ (wherein R' is a hydrogen atom or an organic group),
Y^{f2} is a group composed of one or more selected from the group consisting of an aliphatic group and an aromatic group,
Z^{f} is R^{f1}, or a hydrocarbon group having 2 or more and 40 or less carbon atoms and including R^{f1} or R^{f2}, and
α is 1 to 3].

R^{f1} may be, for example, included in Z^{f}. R^{f2} may be included in an organic group, Y^{f2}, Z^{f} or R^{f2}.

A group reactive with the active hydrogen group in the compound (a), such as acid halide, acid anhydride, carboxylic acid, isocyanate, thioisocyanate, epoxy, halide, amine and hydroxy, may be formed at the end of Y^{f} in -Y^{f}-Z^{f}_{α}. The compound (b) may also be represented by, for example, X^{r}-Y^{f}-Z^{f}_{α}. In the formula, X^{r} forms a group reactive with the active hydrogen group in the compound (a). X^{r} may form, for example, the group independently, or jointly with Y^{f}.

The polymerizable compound (b) may also be a compound represented by

CH₂=C(-X^{f}) -Y^{f}-Z^{f}_{α}

[wherein, independently at each occurrence,
X^{f} is a hydrogen atom, a monovalent organic group or a halogen atom, and
others are as described above]. In the formula, X^{f} is a hydrogen atom, a monovalent organic group or a halogen atom. X^{f} may be a hydrogen atom, a methyl group, a halogen excluding a fluorine atom, a substituted or non-substituted benzyl group, or a substituted or non-substituted phenyl group. It is preferable that X^{f} is a hydrogen atom, a methyl group or a chlorine atom. The polymerizable compound (b) may be an ethylenically unsaturated polymerizable compound such as an acrylic monomer, a styrene monomer or an allyl monomer.

The structure of the compound (b) is not limited to the above structure, and -Y^{f}-Z^{f}_{α} may constitute any moiety of the compound (b) including a compound (b) having an active hydrogen-reactive group or a polymerizable compound (b) .

### (Y^{f})

Y^{f} is a 1+α valent group composed of one or more selected from the group consisting of Y^{f1} and Y^{f2}.

Y^{f1} is a group composed of one or more selected from the group consisting of a direct bond, -O-, -C(=O)-, - C(=NR')-, -S-, -S(=O)₂-, -NR'-, -C(OR')R'-, -C(OR') (-)₂ and -N(-)₂ (wherein R' is a hydrogen atom or a monovalent organic group), and
Y^{f2} is a group composed of one or more selected from the group consisting of an aliphatic group and an aromatic group.

Y^{f} may have a molecular weight of 10 or more, 50 or more, 100 or more, 200 or more, 300 or more, 500 or more, or 750 or more, and 3,000 or less, 2,500 or less, 2,000 or less, 1,500 or less, 1,000 or less, 750 or less, 500 or less, 300 or less, 200 or less, 100 or less, or 50 or less.

In the following, Y^{f1} and Y^{f2} will be described.

### ∘ Y^{f1}

Y^{f1} is a non-hydrocarbon linker.

Y^{f1} is a direct bond or a divalent or higher valent group. Y^{f1} may have a valence of 2 to 4, 2 or 3, or 2. It is preferable that Y^{f1} is not limited to direct bond.

Y^{f1} may have a molecular weight of 10 or more, 50 or more, 100 or more, 200 or more, 300 or more, or 500 or more, and 1,000 or less, 750 or less, 500 or less, 300 or less, 200 or less, 100 or less, or 50 or less.

Y^{f1} is a group composed of one or more selected from -O-, -C(=O)-, -S(=O)₂-, -NR'-, -C(OR')R'- and -C(OR') (-)₂ (wherein R' is independently at each occurrence a hydrogen atom or a monovalent organic group).

R' is a hydrogen atom or a monovalent organic group. The organic group may be an aliphatic group having 1 to 30 carbon atoms (e.g., 1 to 20, 1 to 10, or 1 to 4 carbon atoms) or an aromatic group (e.g., a substituted or non-substituted alkyl group, a substituted or non-substituted benzyl group, or a substituted or non-substituted phenyl group). In the present description, the same applies to other R'.

Examples of Y^{f1} include a direct bond,

-O-,

-O-C(=O)-,

-O-C(=O)-O-,

-O-C(=O)-NR'-,

-NR'-,

-NR'-C(=O)-,

-NR'-C(=O)-O-,

-NR'-C(=O)-NR'-,

-C(=O)-,

-C (=O) -O-,

-C (=O) -NR' -,

-SO₂⁻,

-SO₂NR'-,

-C(OR')R'-,

and

-C(OR')(-)₂,

(wherein R' is independently at each occurrence a hydrogen atom or a monovalent organic group).

### ∘ Y^{f2}

Y^{f2} may be a group composed of one or more selected from the group consisting of an aliphatic group and an aromatic group.

Y^{f2} is a divalent or higher valent group. Y^{f2} may have a valence of 2 to 4, 2 or 3, or 2.

Y^{f2} may have 1 or more, 2 or more, 3 or more, 4 or more, 6 or more, 8 or more, 10 or more, 12 or more, 14 or more, 16 or more, or 18 or more carbon atoms, and 40 or less, 35 or less, 30 or less, 25 or less, 20 or less, 15 or less, 10 or less, or 5 or less carbon atoms.

Y^{f2} may have a molecular weight of 10 or more, 50 or more, 100 or more, 200 or more, 300 or more, or 500 or more, and 2,000 or less, 1,500 or less, 1,000 or less, 750 or less, 500 or less, 300 or less, 200 or less, 100 or less, or 50 or less.

Y^{f2} may be a hydrocarbon group or a non-hydrocarbon group (including a heteroatom). Y^{f2} may be aliphatic or aromatic. Y^{f2} may be linear, branched or cyclic.

Y^{f2} may be a hydrocarbon optionally having a substituent, a hydrocarbon aromatic ring optionally having a substituent, a heterocyclic linker optionally having a substituent, or a combination thereof. For example, Y^{f2} may be composed of one or more selected from a divalent to tetravalent aliphatic hydrocarbon group having 1 to 20 carbon atoms and optionally having a substituent, a divalent to tetravalent hydrocarbon aromatic ring optionally having a substituent, and a divalent to tetravalent heterocyclic ring optionally having a substituent.

The divalent to tetravalent aliphatic hydrocarbon group having 1 to 20 carbon atoms may be a cyclic, branched or linear hydrocarbon group. The divalent to tetravalent aliphatic hydrocarbon group having 1 to 20 carbon atoms may be a saturated or unsaturated (e.g., saturated) aliphatic hydrocarbon group. The aliphatic hydrocarbon group having 1 to 20 carbon atoms may have 1 or more, 2 or more, 3 or more, 4 or more, 6 or more, 8 or more, or 10 or more carbon atoms, and 15 or less, 10 or less, or 5 or less carbon atoms. The aliphatic hydrocarbon group may have a valence of 2 or more, 3 or more, or 4, and 4 or less, 3 or less, or 2.

The aliphatic hydrocarbon group may have a substituent. Examples of the substituents include, for example, -OR', -N(R')₂, -COOR', and a halogen atom (wherein R' is independently at each occurrence a monovalent organic group). The substituent may have or be free of active hydrogen. The number of substituent may be 6 or less, 5 or less, 4 or less, 3 or less, 2 or less, 1 or less, or 0. In the aliphatic hydrocarbon group having a substituent, the amount of carbon atom relative to the amount of carbon atom and heteroatom may be 70 mol% or more, 80 mol% or more, 90 mol% or more, 95 mol% or more, or 99 mol% or more, and is preferably be 75 mol% or more, and may be 95 mol% or less, 90 mol% or less, 85 mol% or less, or 80 mol% or less.

Examples of the divalent to tetravalent hydrocarbon aromatic ring include groups obtained by removing 2 to 4 hydrogen atoms from hydrocarbon aromatic rings such as benzene, naphthalene, anthracene, phenanthrene, tetracene (naphthacene), pentacene, pyrene, and coronene. The number of ring constituting atom of the hydrocarbon aromatic ring is 3 to 20, 4 to 16, or 5 to 12 and preferably 5 to 12. A valence of the hydrocarbon aromatic ring may be 2 or more, 3 or more, or 4, and may be 4 or less, 3 or less, or 2.

The hydrocarbon aromatic ring may have a substituent. Examples of substituents include -R', -OR', -N(R')₂, -COOR' and a halogen atom (wherein R' is independently at each occurrence a hydrogen atom or a monovalent organic group). The substituent may or may not have active hydrogen. The number of substituents may be 6 or less, 5 or less, 4 or less, 3 or less, 2 or less, 1 or less, or 0. In the hydrocarbon aromatic ring having a substituent, the amount of carbon atom relative to the carbon atom and the heteroatom may be 70 mol% or more, 80 mol% or more, 90 mol% or more, 95 mol% or more, or 99 mol% or more, and preferably 75 mol% or more, and may be 95 mol% or less, 90 mol% or less, 85 mol% or less, or 80 mol% or less.

The divalent to tetravalent heterocyclic ring may be an aliphatic group or an aromatic group. Examples of the divalent to tetravalent heterocyclic rings include groups obtained by removing 2 to 4 hydrogen atoms from pyridine, pyrazine, pyrimidine, pyridazine, triazine, quinoline, isoquinoline, quinazoline, cinnoline, phthalazine, quinoxaline, pyrrole, indole, furan, benzofuran, thiophene, benzothiophene, pyrazole, imidazole, benzimidazole, triazole, oxazole, benzoxazole, thiazole, benzothiazole, isothiazole, benzisothiazole, pyrrolidine, piperidine, piperazine, imidazolidine, thiazoline, and the like. The number of ring constituting atom of the heterocyclic ring is 3 to 20, 4 to 16, or 5 to 12 and preferably 5 to 12. A valence of the heterocyclic ring may be 2 or more, 3 or more, or 4, and may be 4 or less, 3 or less, or 2.

The heterocyclic ring may have a substituent. Examples of substituents include -R', -OR', -N(R')₂, -COOR' and a halogen atom (wherein R' is independently at each occurrence a hydrogen atom or a monovalent organic group). The substituent may or may not have active hydrogen. The number of substituents may be 6 or less, 5 or less, 4 or less, 3 or less, 2 or less, 1 or less, or 0. In the heterocyclic ring having a substituent, the amount of carbon atom relative to the carbon atom and the heteroatom may be 60 mol% or more, 70 mol% or more, 80 mol% or more, 90 mol% or more, 95 mol% or more, or 99 mol% or more, and for example 65 mol% or more, and may be 95 mol% or less, 90 mol% or less, 85 mol% or less, 80 mol% or less, or 70 mol% or less.

Examples of Y^{f2} include

-Ali-

-Cy-

-Ali(-)₂

-Cy(-)₂

(-)₂Ali-

(-)₂CY-

(-)₂Ali(-)₂

(-)₂Cy(-)₂

-Ali-Cy-

-Cy-Ali-

-Cy-Ali-Cy-

-Ali-Cy-Ali-

[wherein Ali is an aliphatic hydrocarbon group having 1 to 20 carbon atoms and Cy is a hydrocarbon aromatic ring or a heterocyclic ring].

Specific examples of Y^{f2} include:
- (CH₂)ₚ- (p is 1 to 20, for example, 1 to 10),
   a linear hydrocarbon group having 1 to 40, for example, 1 to 10 carbon atoms and having an unsaturated bond,
   a branched hydrocarbon group having 1 to 40, for example, 1 to 10 carbon atoms, and
- (CH₂)_{q}-Cy-(CH₂)ᵣ- (q and r are each independently 0 to 20, for example, 1 to 10, and Cy is a hydrocarbon aromatic ring or a heterocyclic ring).

### o Examples of Y^{f}

Examples of Y^{f} will be described.

When Y^{f} is divalent, examples of Y^{f} include -Y^{f1}-, - Y^{f1}-Y^{f2}- -Y^{f1}-Y^{f2}-Y^{f1}- -Y^{f1}-Y^{f2}-Y^{f1}-Y^{f2}- -Y^{f2}- -Y^{f2}-Y^{f1}-, - Y^{f2}-Y^{f1}-Y^{f2}- and -y^{f2}-y^{f1}-y^{f2}-y^{f1}-. The group adjacent to Z^{f}_{α} may be Y^{f1},

When Y^{f} is trivalent, examples of Y^{f} include -Y^{f1}(-)₂, -Y^{f1}-Y^{f2}(-)₂, -Y^{f1}-(Y^{f2}-)₂, -Y^{f1}-Y^{f2}-Y^{f1}(-)₂, -Y^{f1}-Y^{f2}(-Y^{f1}-)₂, - Y^{f1}-(Y^{f2}-Y^{f1}-)₂, -Y^{f1}-Y^{f2}-Y^{f1}-Y^{f2}(-)₂, -Y^{f1}-Y^{f2}-Y^{f1}-(Y^{f2}-)₂, -Y^{f1}-Y^{f2}-(Y^{f1}-Y^{f2}-)₂, -Y^{f1}-(Y^{f2}-Y^{f1}-Y^{f2})₂; -Y^{f2}(-)₂, -Y^{f2}-Y^{f1}(-)₂, -Y^{f2}-(Y^{f1}-)₂, -Y^{f2}-Y^{f1}-Y^{f2}(-)₂, -Y^{f2}-Y^{f1}(-Y^{f2}-)₂, -Y^{f2}-(Y^{f1}-Y^{f2}-)₂, -Y^{f2}-Y^{f1}-Y^{f2}-Y^{f1}(-)₂, -Y^{f2}-Y^{f1}-Y^{f2}-(Y^{f1}-)₂, -Y^{f2}-Y^{f1}-(Y^{f2}-Y^{f1}-)₂, and -Y^{f2}-(Y^{f1}-Y^{f2}-Y^{f1}-)₂.

When Y^{f} is tetravalent, examples of Y^{f} include -Y^{f1}(-)₃, -Y^{f1}-Y^{f2}(-)₃, -Y^{f1}-(Y^{f2}-)₃, -Y^{f1}-Y^{f2}-Y^{f1}(-)₃, -Y^{f1}-Y^{f2}(-y^{f1}-)₃, - Y^{f1}-(Y^{f2}-Y^{f1}-)₃, -Y^{f1}-Y^{f2}-Y^{f1}-y^{f2}(-)₃, -Y^{f1}-Y^{f2}-Y^{f1}-(Y^{f2}-)₃, -Y^{f1}-Y^{f2}-(Y^{f1}-Y^{f2}-)₃, -Y^{f1}-(Y^{f2}-Y^{f1}-Y^{f2}-)₃, -Y^{f2}(-)₃, -Y^{f2}-Y^{f1}(-)₃, -Y^{f2}-(Y^{f1}-)₃, -Y^{f2}-Y^{f1}-Y^{f2}(-)₃, -Y^{f2}-Y^{f1}(-Y^{f2}-)₃, -Y^{f2}-(Y^{f2}-Y^{f1}-)₃, -Y^{f2}-Y^{f1}-Y^{f2}-Y^{f1}(-)₃, -Y^{f2}-Y^{f1}-Y^{f2}-(Y^{f1}-)₃, -Y^{f2}-Y^{f1}-(Y^{f2}-Y^{f1}-)₃, and -Y^{f2}-(Y^{f1}-Y^{f2}-Y^{f1}-)₃.

Preferred examples of Y^{f} include -Y^{f1}-, -Y^{f1}-Y^{f2}-, -Y^{f1}-Y^{f2}- Y^{f1}-, -Y^{f1}-Y^{f2}(-)₂, -Y^{f2}-, -Y^{f2}-Y^{f1}-, -Y^{f2}-Y^{f1}-Y^{f2}- and -Y^{f2}-Y^{f1}(-)₂.

### (Z^{f})

Z^{f} is R^{f1}, or a hydrocarbon group having 2 or more and 40 or less carbon atoms and including R^{f1} or R^{f2}. Z^{f} is, for example, The hydrocarbon group including R^{f1} or R^{f2} may be an aromatic hydrocarbon group or an aliphatic hydrocarbon group, and is preferably an aliphatic hydrocarbon group, and in particular a saturated aliphatic hydrocarbon group (an alkyl group). The hydrocarbon group including R^{f1} or R^{f2} is branched or linear, and preferably linear. The hydrocarbon group including R^{f1} or R^{f2} may be saturated or unsaturated. It is preferable that the hydrocarbon group including R^{f1} or R^{f2} is a saturated aliphatic hydrocarbon group (alkyl group). The hydrocarbon group including R^{f1} or R^{f2} may have 2 or more, 4 or more, 6 or more, 8 or more, 10 or more, 12 or more, 14 or more, 16 or more, 18 or more, 20 or more, or 22 or more carbon atoms, preferably 10 or more, 12 or more, 14 or more, or 16 or more carbon atoms, and may have 40 or less, 35 or less, 30 or less, 25 or less, 20 or less, 15 or less, or 10 or less carbon atoms, and preferably 30 or less, 25 or less or 20 or less carbon atoms.

### (α)

α is 1 to 3, (1, 2 or 3) and for example, 1 or 2, or 1.

### (Examples of Compound (b))

The compound (b) may have a group represented by the formula:

-Y^{f11}-(Y^{f21}-Y^{f12)}_{β-}Y^{f22-}Y^{f13}-Z^{f}

[wherein, independently at each occurrence,
Y^{f11} is a direct bond, -O-, -NH-, -C(=O)-, -C(=O)-NH- or -S-,
Y^{f21} is a group composed of one or more selected from the group consisting of an aliphatic group and an aromatic group,
Y^{f12} is a group composed of one or more selected from -O-, -C(=O)-, -C(=NR')-, -S-, -S(=O)₂-, -NR'- and - C(OR')R'- (wherein R' is a hydrogen atom or an organic group),
β is 0 to 3,
Y^{f22} is a group composed of one or more selected from the group consisting of an aliphatic group and an aromatic group,
Y^{f13} is -O-, -O-C(=O)-O-, -O-C(=O)-NR'-, -NR'-, -NR'-C(=O)-O-, -NR'-C(=O)-NR'-, -C(=O)-O-, -C(=O)-NR'- or - SO₂NR'- (wherein R' is a hydrogen atom or an organic group), and
Z^{f} is R^{f1}, or a hydrocarbon group having 2 or more and 40 or less carbon atoms and including R^{f1} or R^{f2}].

An active hydrogen-reactive group such as acid halide, acid anhydride, isocyanate and epoxy may be formed at the end of Y^{f11}.

Y^{f11} is a direct bond, -O-, -NH-, -C(=O)-, -C(=O)-NH- or -S-.

Y^{f21} may be a group composed of one or more selected from the group consisting of an aliphatic group and an aromatic group. The same explanation about the above Y^{f2} may apply. Y^{f21}, however, is a divalent group.

Y^{f12} may be a group composed of one or more selected from -O-, -C(=O)-, -C(=NR')-, -S-, -S(=O)₂-, -NR'- and - C(OR')R'-. The same explanation about the above Y^{f1} may apply. Y^{f12}, however, is a divalent group.

β is 0 to 3 (0, 1, 2 or 3) and for example, 0 to 2, in particular 0 or 1.

Y^{f22} may be a group composed of one or more selected from the group consisting of an aliphatic group and an aromatic group. The same explanation about the above Y² may apply. Y^{f22}, however, is a divalent group. Y^{f22} may be a group represented by the formula:

-Y^{f221}-Y^{f222}-

[wherein
Yf²²¹ is a direct bond or a hydrocarbon group having 2 to 40 carbon atoms, and
Y^{f222} is a direct bond or a phenylene group]. The hydrocarbon group may be an aromatic hydrocarbon group or an aliphatic hydrocarbon group, and may be an aliphatic hydrocarbon group, and in particular a saturated aliphatic hydrocarbon group (an alkyl group). The hydrocarbon group is branched or linear, and for example, linear. The hydrocarbon group may be saturated or unsaturated. The hydrocarbon group may be a saturated aliphatic hydrocarbon group (alkyl group). The hydrocarbon group may have 2 or more, 4 or more, 6 or more, 8 or more, 10 or more, 12 or more, 14 or more, 16 or more, 18 or more, 20 or more, or 22 or more carbon atoms, and 40 or less, 35 or less, 30 or less, 25 or less, 20 or less, 15 or less, or 10 or less carbon atoms.

Y^{f13} may be -O-, -O-C(=O)-O-, -O-C(=O)-NR'-, -NR'-, - NR'-C(=O)-O-, -NR'-C(=O)-NR'-, -C(=O)-O-, -C(=O)-NR'- or -SO₂NR'- (wherein R' is a hydrogen atom or a monovalent organic group). Y^{f13} is, for example, -O- or -NR'-.

Z^{f} is R^{f1}, or a hydrocarbon group having 2 or more and 40 or less carbon atoms and including R^{f1} or R^{f2} as described above. In particular, Z^{f} is

Alternatively, when the compound (b) is polymerizable, the compound (b) may be an acrylic monomer or an allyl monomer represented by

CH₂=C(-X^{f})-C(=O)-Y^{f11}-(Y^{f21}-Y^{f12})_{β}-Y^{f22}-Y^{f13-}Z^{f}

or

CH₂=C(-X^{f})-CH₂-Y^{f11}-(Y^{f21}-Y^{f12})_{β}-Y^{f22}-Y^{f13}-Z^{f}

[wherein, independently at each occurrence,
X^{f} is a hydrogen atom, a monovalent organic group or a halogen atom, and others are as described above].

In an embodiment, Y^{f22} may be a group represented by the formula:

-Y^{f221}-Y^{f222}-

[wherein
y^{f221} is a direct bond or a hydrocarbon group having 2 to 40 carbon atoms, and
Y^{f222} is a direct bond or a phenylene group].
Y^{f113} may be -O- or -NR'- (wherein R' is a hydrogen atom or a monovalent organic group). β may be 0 or 1. Z^{f} may be

### (Specific examples of compound (b))

Specific examples of compounds (b) are as follows.

| | |
|---|---|
| Acid halide | G(O=)C-Y^{f}-Z^{f}_{α} |
| Acid anhydride | O(C(=O)-Y^{f}-Z^{f}_{α})2 |
| Carboxylic acid | HO(O=)C-Y^{f}-Z^{f}_{α} |
| Isocyanate | O=C=N-Y^{f-}Z^{f}_{α} |
| Thioisocyanate | S=C=N-Y^{f}-Z^{f}_{α} |
| Epoxy | (CH₂OCH)CH₂O-Y^{f}-Z^{f}_{α} |
| Halide | G-Y^{f}-Z^{f}_{α} |
| Amine | H₂N-Y^{f}-Z^{f}_{α} |
| Hydroxy | HO-Y^{f}-Z^{f}_{α} |

[wherein Y^{f}, Z^{f} and α are as described above, and G is a halogen atom (e.g., F, Cl, Br, or I)].

In the above formula, the terminal functional groups (e.g., G(O=)C) adjacent to Y^{f}-Z^{f}_{α} are shown as being not included in -Y^{f}-, but part of the structure of the terminal functional groups (e.g., (O=)C) may be re-written to be incorporated into Y^{f}.

Examples of compounds (b) also include a polymerizable compound (b) and a polymer thereof, and the polymerizable compound (b) is prepared by reacting one of the compounds listed above as specific examples with a compound having an ethylenically unsaturated polymerizable group (such as (meth)acryloyl chloride, 2-((meth)acryloyloxy)ethyl isocyanate, glycidyl (meth)acrylate, allyl glycidyl ether, glycidyl α-ethyl acrylate, crotonylglycidyl ether, (iso)crotonic acid glycidyl ether, (3,4-epoxycyclohexyl)methyl (meth)acrylate, (meth)acrylic acid chloride, (meth)acryloyl chloride, (meth)acrylamide, (meth)acrylic acid, N,N-dimethylaminoethyl (meth)acrylate, 2-hydroxyethyl (meth)acrylate, allylamine and allyl alcohol).

Specific examples of compounds (b) include a compound in which -Y^{f}-Z^{f}_{α} in the formula shown as the above examples is

-Ali-O-R^{f1},

-Ali-Cy-O-R^{f1},

-Ali-Cy-Ali-O-R^{f1},

-Cy-O-R^{f1},

-Cy-Ali-O-R^{f1},

-Cy-Ali-Cy-O-R^{f1},

-Ali-N(R')-R^{f1},

-Ali-Cy-N(R')-R^{f1},

-Ali-Cy-Ali-N(R')-R^{f1},

-Cy-N(R')-R^{f1},

-Cy-Ali-N(R')-R^{f1},

-Cy-Ali-Cy-N(R')-R^{f1},

[wherein Ali is an aliphatic hydrocarbon group having 1 to 20 carbon atoms, Cy is a hydrocarbon aromatic ring or a heterocyclic ring, R' is a hydrogen atom or an organic group, and R^{f1} is -CF₃, -CF₂H or -CFH₂].

Specific examples of compounds (b) also include a compound in which -Y^{f}-Z^{f}_{α} in the formula shown as the above examples is

-(CH₂)ₙ-O-R^{f1},

-(CH₂)ₙ-Cy-O-R^{f1},

-(CH₂)ₙ-Cy-(CH₂)ₙ-O-R^{f1},

-Cy-O-R^{f1},

-Cy-(CH₂)ₙ-O-R^{f1},

-Cy-(CH₂)ₙ-Cy-O-R^{f1},

-(CH₂)ₙ-N(R')-R^{f1},

-(CH₂)ₙ-Cy-N(R')-R^{f1},

- (CH₂)ₙ-Cy-(CH₂) ₙ-N(R')-R^{f1},

-Cy-N(R')-R^{f1},

-Cy-(CH₂)ₙ-N(R')-R^{f1},

-Cy-(CH₂)ₙ-Cy-N(R')-R^{f1},

[wherein n is an integer of 1 to 40, Cy is a hydrocarbon aromatic ring or a heterocyclic ring, R' is a hydrogen atom or an organic group, R^{f1} is -CF₃, -CF₂H or -CFH₂].

More specific examples of compounds (b) include: trifluoromethoxyalkylcarboxylic acid, trifluoromethoxyalkylcarboxylic acid halide, trifluoromethoxybenzoic acid, trifluoromethoxy benzoic acid halide, trifluoromethoxyphenylacetic acid, trifluoromethoxy phenylacetic acid halide, trifluoromethoxy alkylamine, trifluoromethoxy aniline, trifluoromethoxy alkyl alcohol, trifluoromethoxy phenol, trifluoromethoxy alkyl isocyanate, CF₃-O-CF₂-R²-OH CF₃-O-R¹-O-CF₂-R²-OH CF₃-O-R¹-CF₂-O-R²-OH CF₃-O-Ph-O-CF₂-R²-OH CF₃-O-Ph-CF₂-O-R²-OH R³-O-CF₂-R²-OH R³-CF₂-O-R²-OH (wherein R¹ to R³ are independently a hydrocarbon group having 1 to 40 carbon atoms).

### [Examples of method for producing compound (b)]

In this method, compound (111): HO-R^{j33}-CH=CH₂ having a hydroxyl group and olefin is used as a raw material to synthesize a trifluoromethoxy group-containing compound. Rⱼ₃₃ is an alkylene group, for example, a C1-30 alkylene group.

Examples of olefin group-containing compounds include 3-hydroxypropene, 4-hydroxy-1-butene, 5-hydroxy-1-pentene, 6-hydroxy-1-hexene, 7-hydroxy-1-heptene, 8-hydroxy-1-octene, 9-hydroxy-1-nonene, 10-hydroxy-1-decane, 11-hydroxy-1-undecane, 12-hydroxy-1-dodecane, 13-hydroxy-1-tridecane, 14-hydroxy-1-tetradecane, 15-hydroxy-1-pentadecane, 16-hydroxy-1-hexadecane, 17-hydroxy-1-heptadecane, 18-hydroxy-1-octadecane, 19-hydroxy-1-nonadecane, 20-hydroxy-1-icosane, 21-hydroxy-1-henicosane, 24-hydroxy-1-tetracosane and 30-hydroxy-1-triacontane.

Carbon disulfide is allowed to react with the hydroxyl group of the compound (111) in the presence of, for example, sodium hydride, and iodomethane is added thereto to give xanthate ester (112): H₃CS-C(=S)-O-R^{j33}-COOR^{j32}. R^{j32} is a lower alkylene group such as a methyl group, an ethyl group, a propyl group, a butyl group or a tert-butyl group, and more specifically a methyl group or a tert-butyl group. Pyridine hydrogen fluoride and 1,3-dibromo-5,5-dimethyl hydantoin are allowed to react with the above xanthate ester (102) to give the target trifluoromethoxy group-containing compound (113): F₃CO-Rⱼ₃₃-CH=CH₂.

In another method, the hydroxyl group of the compound (111) is allowed to react with trifluoromethyl arylsulfonate in an aprotic polar solvent such as NMP, DMF or HMPA (hexamethylphosphoric triamide) in the presence of cesium fluoride and/or tetrabutylammonium bromide to give the target trifluoromethoxy group-containing compound (113): F₃CO-Rⱼ₃₃-CH=CH₂.

The resulting F₃CO-Rⱼ₃₃-CH=CH₂ may be formed into F₃CORⱼ₃₃CH₂CH₂-OH by a usual method. The resultant may also be allowed to react with a compound having an ethylenically unsaturated polymerizable group. For example, a (meth)acrylate monomer may be synthesized by the reaction with (meth)acrylic acid. A (meth)acrylate monomer containing a urethane group may be synthesized by the reaction with (meth)acrylate containing an isocyanate group. Alcohol and methyl acrylate may be allowed to react in the presence of a basic catalyst (e.g., calcium hydroxide) to give fluorine-containing acrylate.

In this regard, another method for producing F₃CORⱼ₃₃CH₂CH₂-OH is a method described in, for example, WO2020/168011A1, in which F₃CO-Rⱼ₃₃CH₂CH₂-OH is synthesized by reacting silver triflate, potassium fluoride, 1-(chloromethyl)-4-fluoro-4-diazoniabicyclo[2.2.2]octane, a salt of tetrafluoroborate and benzyloxy alcohol (e.g., benzyloxy ethanol) and reducing the resultant (e.g., [2-(trifluoromethoxy)ethoxy]methyl]benzene) with hydrogen in the presence of a palladium catalyst. For F₃CO-Rⱼ₃₃CH₂CH₂-OH, a (meth)acrylate monomer may also be synthesized by the reaction with (meth)acrylic acid. A (meth)acrylate monomer containing a urethane group may be synthesized by the reaction with (meth)acrylate containing an isocyanate group. Alcohol and methyl acrylate may be allowed to react in the presence of a basic catalyst (e.g., calcium hydroxide) to give fluorine-containing acrylate.

### [Other moieties]

The fluorine-containing compound may also have a modifying group for the compound (a) (e.g., a modifying group other than -Y^{f}-Z^{f}ₙ) in addition to the structures described above. Examples of modifying groups include an anionic group, a cationic group and a monovalent hydrocarbon group 2 or more and 40 or less carbon atoms.

Examples of anionic groups include a monomer having a carboxyl group, a sulfonic acid group or a phosphoric acid group.

Examples of salts of the anionic group include alkaline metal salt, alkaline earth metal salt, and an ammonium salt such as methyl ammonium salt, ethanol ammonium salt and triethanol ammonium salt.

Examples of cationic groups include an amino group, which is preferably a tertiary amino group and a quaternary amino group. It is preferable that in the tertiary amino group, two groups bonded to a nitrogen atom, which are the same or different, are an aliphatic group having 1 to 5 carbon atoms (in particular alkyl group), an aromatic group having 6 to 20 carbon atoms (an aryl group), or an aromatic aliphatic group having 7 to 25 carbon atoms (in particular an aralkyl group, e.g., a benzyl group (C₆H₅-CH₂-)). It is preferable that in the quaternary amino group, three groups bonded to a nitrogen atom, which are the same or different, are an aliphatic group having 1 to 5 carbon atoms (in particular alkyl group), an aromatic group having 6 to 20 carbon atoms (an aryl group), or an aromatic aliphatic group having 7 to 25 carbon atoms (in particular an aralkyl group, e.g., benzyl group (C₆H₅-CH₂-)). In the tertiary amino group and the quaternary amino group, the last group bonded to the nitrogen atom may have a carbon-carbon double bond. The cationic group may be in the form of salt.

The cationic group in the form of salt is a salt with an acid (an organic acid or an inorganic acid). An organic acid such as a carboxylic acid having 1 to 20 carbon atoms (in particular, a monocarboxylic acid such as acetic acid, propionic acid, butyric acid and stearic acid) are preferred.

The monovalent hydrocarbon group may be an aromatic hydrocarbon group or an aliphatic hydrocarbon group, and may be an aliphatic hydrocarbon group, and in particular a saturated aliphatic hydrocarbon group (an alkyl group). The hydrocarbon group is branched or linear, and for example, linear. The hydrocarbon group may be saturated or unsaturated. The hydrocarbon group may be a saturated aliphatic hydrocarbon group (alkyl group). The hydrocarbon group may have 2 or more, 4 or more, 6 or more, 8 or more, 10 or more, 12 or more, 14 or more, 16 or more, 18 or more, 20 or more, or 22 or more carbon atoms, and 40 or less, 35 or less, 30 or less, 25 or less, 20 or less, 15 or less, or 10 or less carbon atoms.

### {Method for producing fluorine-containing compound}

The fluorine-containing compound may be produced by reacting the compound (a) and the compound (b). The reaction conditions may be suitably determined by those skilled in the art. The active hydrogen that the compound (a) has and the active hydrogen-reactive group that the compound (b) has may be reacted to form a fluorine-containing compound. Alternatively, the compound (a) is graft-polymerized with the compound (b) using an appropriate catalyst (e.g., a polyvalent ion selected from Ce⁴⁺, V⁵⁺, Cr⁶⁺ and Mn³⁺) to form a fluorine-containing compound. The grafting-to method, the grafting-from method, and the like, which are known to those skilled in the art, may be used. For the graft polymerization, for example, textbook "Principles of Polymerization", G. G. Odian, Wiley Interscience, 1991, third edition, pp. 715-725 may be referred to.

### <Water- and oil- repellent agent>

The water- and oil-repellent agent in the present disclosure imparts liquid-repellency (water-repellency, oil-repellency, oil resistance and/or water resistance) to a substrate (e.g., fiber substrate, paper substrate), and may function as at least one selected from a water-repellent agent, an oil-repellent agent, an oil-resistant agent and a water-resistant agent. The water- and oil-repellent agent in the present disclosure can impart good oil resistance (oil-repellency) and/or water resistance (water-repellency) to a substrate (e.g., fiber substrate, paper substrate), and for example, can impart both oil resistance and water resistance to a substrate.

### {Fluorine-containing compound}

The water- and oil-repellent agent of present disclosure comprises the fluorine-containing compound described above. The fluorine-containing compound itself may be used as a water- and oil-repellent agent, or may be used as a water- and oil-repellent agent in combination with other components described below.

The water- and oil-repellent agent in the present disclosure comprises the fluorine-containing-compound having R^{f1} or R^{f2} described above. Meanwhile, the water- and oil-repellent agent in the present disclosure may not have any one selected from the group consisting of a compound having a fluoroalkyl group having 8 or more carbon atoms, a compound having a perfluoroalkyl group having 8 or more carbon atoms, a compound having a fluoroalkyl group having 4 or more carbon atoms, a compound having a perfluoroalkyl group having 4 or more carbon atoms, a compound having a fluoroalkyl group having 2 or more carbon atoms and a compound having a perfluoroalkyl group having 2 or more carbon atoms. The water- and oil-repellent agent in the present disclosure can impart liquid-repellency to a substrate without including the above fluorine compounds. As used herein, the fluoroalkyl group may mean an alkyl group in which one or more hydrogen atoms at the respective carbon atoms are substituted by a fluorine atom.

### [Amount of fluorine-containing compound]

The amount of the fluorine-containing compound may be 0.01% by weight or more, 0.03% by weight or more, 0.5% by weight or more, 1% by weight or more, 3% by weight or more, 5% by weight or more, 10% by weight or more, 20% by weight or more, 30% by weight or more, and 60% by weight or less, 50% by weight or less, 40% by weight or less, 30% by weight or less, 20% by weight or less, 10% by weight or less, 5% by weight or less, or 3% by weight or less in the water- and oil-repellent agent. The fluorine-containing compound itself may be used as a water- and oil-repellent agent.

### {Other water- and oil- repellent components}

The water- and oil-repellent agent of present disclosure may comprise other water- and oil-repellent components. A known water- and oil-repellent agent may be used as the other water- and oil-repellent agent, and for example, a non-fluorine water- and oil-repellent agent may be used. Examples of non-fluorine water- and oil-repellent components include, but are not limited to, acrylic polymers described, for example, in WO/2020/054856, WO/2017/159754 and WO/2020/162547, polyurethane described in, for example, National Publication of International Patent Application No. 2016-524628, National Publication of International Patent Application No. 2017-533976, National Publication of International Patent Application No. 2017-504730, WO/2021/132170 and WO/2021/132172, and a modified natural product described, for example, in WO/2022/065382 and WO/2022/065385.

### [Amount of other water- and oil- repellent components]

The amount of other water- and oil- repellent components may be 0.01 parts by weight or more, 0.1 parts by weight or more, 1 part by weight or more, 3 parts by weight or more, 5 parts by weight or more, 10 parts by weight or more, 15 parts by weight or more, 20 parts by weight or more, 50 parts by weight or more, 75 parts by weight or more, or 100 parts by weight or more, and 500 parts by weight or less, 300 parts by weight or less, 200 parts by weight or less, 100 parts by weight or less, 30 parts by weight or less, 20 parts by weight or less, 10 parts by weight or less, 5 parts by weight or less, 3 parts by weight or less, or 1 part by weight or less relative to 100 parts by weight of the fluorine-containing compound.

### {Dispersant}

The water- and oil-repellent agent of present disclosure may comprise a dispersant.

The dispersant may be at least one selected from an organic dispersant and an inorganic dispersant. The dispersant may be non-anionic, and may be at least one selected from a nonionic dispersant, a cationic dispersant, an amphoteric dispersant and an inorganic dispersant. The water- and oil-repellent agent may not include an anionic dispersant.

An organic dispersant and an inorganic dispersant may be used as the dispersant, respectively, or an organic dispersant and an inorganic dispersant may be used in combination.

An organic dispersant may be used as the dispersant. The organic dispersant may be classified into a nonionic dispersant, an anionic dispersant, a cationic dispersant and an amphoteric dispersant. The organic dispersant may mean a surfactant.

The dispersant may have no fluorine.

### [Nonionic dispersant]

The dispersant may comprise a nonionic dispersant. The nonionic dispersant may be a nonionic surfactant.

The nonionic dispersant may be of low molecular weight or high molecular weight. The nonionic dispersant may have a molecular weight of 100 or more, 500 or more, 1,000 or more, 2,000 or more, 4,000 or more, or 6,000 or more, and 100,000 or less, 50,000 or less, 25,000 or less, 10,000 or less, 7,500 or less, 5,000 or less, 2,500 or less, 1,000 or less, 750 or less, or 250 or less.

Examples of nonionic dispersant include ether, ester, ester ether, alkanolamide, polyol and amine oxide.

The ether is, for example, a compound having an oxyalkylene group (preferably a polyoxyethylene group).

The ester is, for example, an ester of an alcohol and a fatty acid. The alcohol is, for example, an alcohol which is monohydric to hexahydric (particularly dihydric to pentahydric) and has 1 to 50 carbon atoms (particularly 10 to 30 carbon atoms) (for example, an aliphatic alcohol) . Examples of the fatty acids are saturated or unsaturated fatty acids having 2 to 50 carbon atoms, particularly 5 to 30 carbon atoms.

The ester ether is, for example, a compound in which an alkylene oxide (particularly ethylene oxide) is added to an ester of an alcohol and a fatty acid. The alcohol is, for example, an alcohol which is monohydric to hexahydric (particularly dihydric to pentahydric) and has 1 to 50 carbon atoms (particularly 3 to 30 carbon atoms) (for example, an aliphatic alcohol). Examples of the fatty acids are saturated or unsaturated fatty acids having 2 to 50 carbon atoms, particularly 5 to 30 carbon atoms.

The alkanolamide is formed of for example, a fatty acid and an alkanolamine. The alkanolamide may be a monoalkanolamide or a dialkanolamide. Examples of the fatty acids are saturated or unsaturated fatty acids having 2 to 50 carbon atoms, particularly 5 to 30 carbon atoms. The alkanolamine may be an alkanol with 1 to 3 amino groups and 1 to 5 hydroxyl groups, having 2 to 50, particularly 5 to 30 carbon atoms.

The polyol may be, for example, a dihydric to pentahydric alcohol having 10 to 30 carbon atoms.

The amine oxide may be an oxide (for example, having 5 to 50 carbon atoms) of an amine (secondary amine or preferably tertiary amine).

The nonionic dispersant is preferably a nonionic dispersant having an oxyalkylene group (preferably a polyoxyethylene group). The alkylene group in the oxyalkylene group preferably has 2 to 10 carbon atoms. The number of oxyalkylene groups in the molecule of the nonionic dispersant is generally preferably 2 to 100.

The nonionic dispersant is selected from the group consisting of an ether, an ester, an ester ether, an alkanolamide, a polyol, or an amine oxide, and is preferably a nonionic dispersant having an oxyalkylene group.

The nonionic dispersant may be, for example, an alkylene oxide adduct of a linear and/or branched aliphatic (saturated and/or unsaturated) group, a polyalkylene glycol ester of a linear and/or branched fatty acid (saturated and/or unsaturated), a sorbitan ester of a linear and/or branched fatty acid (saturated and/or unsaturated), a glycerin ester of a linear and/or branched fatty acid (saturated and/or unsaturated), a polyglycerol ester of a linear and/or branched fatty acid (saturated and/or unsaturated), a sucrose ester of a linear and/or branched fatty acid (saturated and/or unsaturated), a polyoxyethylene (POE)/polyoxypropylene (POP) copolymer (random copolymer or block copolymer), and an alkylene oxide adduct of acetylene glycol. Among them, the nonionic dispersant is preferably a dispersant such that the structures of the alkylene oxide addition moiety and polyalkylene glycol moiety are polyoxyethylene (POE) or polyoxypropylene (POP) or POE/POP copolymer (which may be a random or block copolymer, for example.).

Furthermore, the nonionic dispersant may not include an aromatic group.

The nonionic dispersant may be the compound represented by the formula:

R¹O-(CH₂CH₂O)ₚ-(R²O)_{q}-R³

[wherein R¹ is an alkyl group having 1 to 22 carbon atoms, an alkenyl group or an acyl group, having 2 to 22 carbon atoms,
R² is each independently the same or different and is an alkylene group having 3 or more carbon atoms (for example, 3 to 10),
R³ is a hydrogen atom, an alkyl group having 1 to 22 carbon atoms, or an alkenyl group having 2 to 22 carbon atoms,
p is a numeral of 2 or more,
q is 0 or a numeral of 1 or more.].

R¹ preferably has 8 to 20 carbon atoms, particularly 10 to 18 carbon atoms. Preferred specific examples of R¹ include a lauryl group, a tridecyl group, and an oleyl group.

R² is, for example, a propylene group and a butylene group.

In the nonionic dispersant, for example, p may be a numeral of 3 or more (for example, 5 to 200) and q may be a numeral of 2 or more (for example, 5 to 200). Namely, - (R²O)_{q}- may form, for example, a polyoxyalkylene chain.

The nonionic dispersant may be, for example, a polyoxyethylene alkylene alkyl ether comprising a hydrophilic polyoxyethylene chain and a hydrophobic oxyalkylene chain (particularly a polyoxyalkylene chain) in the center. The hydrophobic oxyalkylene chain includes, for example, an oxypropylene chain, an oxybutylene chain, and a styrene chain. The oxypropylene chain is preferred among them.

Specific examples of the nonionic surfactants include a condensation product of ethylene oxide with hexylphenol, isooctatylphenol, hexadecanol, oleic acid, an alkane(C₁₂-C₁₆) thiol, a sorbitan monofatty acid (C₇-C₁₉), an alkyl (C₁₂-C₁₈)amine, or the like, and a sorbitan fatty acid ester, a glycerin fatty acid ester, a polyglycerin fatty acid ester, a sucrose fatty acid ester, a propylene glycol fatty acid ester, a polyoxyethylene alkyl ether, a polyoxyethylene polyoxypropylene alkyl ether, a polyoxyethylene glycerin fatty acid ester, a polyoxyethylene sorbitan fatty acid ester, and a lecithin derivative.

The proportion of the polyoxyethylene block can be 5 to 80% by weight, for example, 30 to 75% by weight, particularly 40 to 70% by weight, based on a molecular weight of the nonionic dispersant (copolymer).

The average molecular weight of the nonionic dispersant is generally 300 to 5,000, for example, 500 to 3,000.

For example, the nonionic dispersant may be used singly or in admixture of two or more. The nonionic dispersant may be a mixture of a compound with an HLB (hydrophilic-hydrophobic balance) of less than 15 (particularly 5 or less) and a compound with an HLB of 15 or more.

### [Cationic dispersant]

The dispersant may comprise a cationic dispersant. The cationic dispersant may be a cationic surfactant. The cationic dispersant may be of low molecular weight (with a molecular weight of 2,000 or less, in particular, 10,000 or less) or of high molecular weight (with a molecular weight of, for example, 2,000 or more). The cationic dispersant may not have an amide group.

The cationic dispersant may be of low molecular weight or high molecular weight. The cationic dispersant may have a molecular weight of 100 or more, 500 or more, 1,000 or more, 2,000 or more, 4,000 or more, or 6,000 or more, and 100,000 or less, 50,000 or less, 25,000 or less, 10,000 or less, 7,500 or less, 5,000 or less, 2,500 or less, 1,000 or less, 750 or less, or 250 or less.

The cationic dispersant may be an amine salt, quaternary ammonium salt, or oxyethylene-added ammonium salt. Specific examples of the cationic surfactant include, but are not limited to, amine salt type dispersants such as an alkylamine salt, amino alcohol fatty acid derivative, polyamine fatty acid derivative, and imidazoline; quaternary ammonium salt type dispersants such as an alkyltrimethylammonium salt, dialkyldimethylammonium salt, alkyldimethylbenzylammonium salt, pyridinium salt, alkylisoquinolinium salt, benzalkonium chloride and benzethonium chloride.

Preferred examples of the cationic dispersant are the compound represented by the formula:

R²¹-N⁺(-R²²)(-R²³)(-R²⁴)X-

[wherein R²¹, R²², R²³, R²⁴ are each a hydrocarbon group having 1 to 40 carbon atoms, and
X is an anionic group.].

Specific examples of R²¹, R²², R²³, -R²⁴ are alkyl groups (for example, a methyl group, a butyl group, a stearyl group, and a palmityl group). Specific examples of X are halogen (for example, chlorine) and acids (hydrochloric acid and acetic acid).

The cationic dispersant is particularly preferably a monoalkyltrimethylammonium salt (with an alkyl having 4 to 40 carbon atoms).

The cationic dispersant is preferably an ammonium salt. The cationic dispersant may be, for example, an ammonium salt represented by the formula:

R¹p-N⁺R²_{q}X-

[wherein R¹ is a C12 or more (for example C₁₂ to C₅₀) linear and/or branched aliphatic (saturated and/or unsaturated) group,
R² is H or a C1 to C4 alkyl group, a benzyl group, a polyoxyethylene group (the number of oxyethylene group is, for example, 1 (particularly 2, especially 3) to 50),
and is particularly preferably CH₃ and C₂H₅,
X is a halogen atom (for example) and a C₁ to C₄ fatty acid base,
p is 1 or 2, q is 2 or 3, and p + q=4.].
The number of carbon atoms of R¹ may be 12 to 50, for example, 12 to 30.

Specific examples of the cationic dispersants include dodecyltrimethylammonium acetate, trimethyltetradecylammonium chloride, hexadecyltrimethylammonium bromide, trimethyloctadecylammonium chloride, (dodecylmethylbenzyl)trimethylammonium chloride, benzyldodecyldimethylammonium chloride, methyldodecyl di(hydropolyoxyethylene) ammonium chloride, benzyldodecyl di(hydropolyoxyethylene) ammonium chloride, and N-[2-(diethylamino)ethyl]oleamide hydrochloride.

### [Anionic dispersant]

The dispersant may comprise an anionic dispersant. The anionic dispersant may be an anionic surfactant. The dispersant may not include an anionic dispersant.

The anionic dispersant may be of low molecular weight or high molecular weight. The anionic dispersant may have a molecular weight of 100 or more, 500 or more, 1,000 or more, 2,000 or more, 4,000 or more, or 6,000 or more, and 100,000 or less, 50,000 or less, 25,000 or less, 10,000 or less, 7,500 or less, 5,000 or less, 2,500 or less, 1,000 or less, 750 or less, or 250 or less.

Examples of the anionic surfactant include an alkyl ether sulfate, an alkyl sulfate, an alkenyl ether sulfate, an alkenyl sulfate, an olefin sulfonate, an alkanesulfonate, a saturated or unsaturated fatty acid salt, an alkyl or alkenyl ether carbonate, an α-sulfone fatty acid salt, a N-acylamino acid dispersant, a phosphate mono- or diester dispersant, and a sulfosuccinic acid ester.

### [Amphoteric dispersant]

The dispersant may comprise an amphoteric dispersant. The amphoteric dispersant may be an amphoteric surfactant.

The amphoteric dispersant may be of low molecular weight or high molecular weight. The amphoteric dispersant may have a molecular weight of 100 or more, 500 or more, 1,000 or more, 2,000 or more, 4,000 or more, or 6,000 or more, and 100,000 or less, 50,000 or less, 25,000 or less, 10,000 or less, 7,500 or less, 5,000 or less, 2,500 or less, 1,000 or less, 750 or less, or 250 or less.

Examples of the amphoteric dispersants include, for example, alanines, imidazolinium betaines, amidobetaines, and acetic acid betaine, and specific examples of the amphoteric dispersants include, for example, lauryl betaine, stearyl betaine, lauryl carboxymethyl hydroxyethyl imidazolinium betaine, lauryl dimethylamino acetic acid betaine, and fatty acid amidopropyldimethylaminoacetic acid betaine.

### [Inorganic dispersant]

The dispersant may comprise an inorganic dispersant.

The inorganic dispersant has an average primary particle size of 5 nm or larger, 30 nm or larger, 100 nm or larger, 1 µm or larger, 10 µm or larger, or 25 µm or larger, and 100 µm or smaller, 50 µm or smaller, 10 µm or smaller, 1 µm or smaller, 500 nm or smaller, or 300 nm or smaller. The average primary particle size may be measured by a microscope, for example, a scanning electron microscope or a transmission electron microscope. The inorganic dispersant may be hydrophilic particles.

Examples of inorganic dispersants include polyvalent metal phosphate such as tricalcium phosphate, magnesium phosphate, aluminum phosphate, zinc phosphate and hydroxyapatite; carbonate such as calcium carbonate and magnesium carbonate; silicate such as calcium metasilicate; sulfate such as calcium sulfate and barium sulfate; and hydroxide such as calcium hydroxide, magnesium hydroxide and aluminum hydroxide.

### [Amount of Dispersant]

The amount of dispersant may be 0.01 parts by weight or more, 0.1 parts by weight or more, 1 part by weight or more, 3 parts by weight or more, 5 parts by weight or more, 10 parts by weight, 15 parts by weight or more, 20 parts by weight or more, 50 parts by weight or more, 75 parts by weight or more, or 100 parts by weight or more, relative to 100 parts by weight of the fluorine-containing compound, and may be 500 parts by weight or less, 300 parts by weight or less, 200 parts by weight or less, 100 parts by weight or less 30 parts by weight or less, 20 parts by weight or less, 10 parts by weight or less, 5 parts by weight or less, 3 parts by weight or less, or 1 part by weight or less.

### {Liquid medium}

The water- and oil-repellent agent in the present disclosure may comprise a liquid medium. The liquid medium may be water, an organic solvent, or a mixture of water and an organic solvent. The water- and oil-repellent agent may be a dispersion or a solution. The water- and oil-repellent agent in the present disclosure may be in the form of a water dispersion, and may include at least water.

Examples of the organic solvents include esters (for example, esters having 2 to 40 carbon atoms, specifically ethyl acetate and butyl acetate), ketones (for example, ketones having 2 to 40 carbon atoms, specifically methyl ethyl ketone and diisobutyl ketone), alcohols (for example, alcohols having 1 to 40 carbon atoms, specifically isopropyl alcohol), aromatic solvents (for example, toluene and xylene), petroleum-based solvents (for example, alkanes having 5 to 10 carbon atoms, specifically, naphtha and kerosene). The organic solvent is preferably a water-soluble organic solvent. The water-soluble organic solvent may include a compound having at least one hydroxy group (for example, polyol such as alcohol and glycol solvent, and an ether form of polyol (for example, a monoether form)). These may be used alone, or two or more of them may be used in combination.

### [Amount of liquid medium]

The amount of liquid medium may be 1 part by weight or more, 3 parts by weight or more, 5 parts by weight or more, 10 parts by weight or more, 20 parts by weight or more, 30 parts by weight or more, 40 parts by weight or more, or 50 parts by weight or more, 100 parts by weight or more, 200 parts by weight or more, 300 parts by weight or more, 500 parts by weight or more, or 1,000 parts by weight or more, and 3,000 parts by weight or less, 2,000 parts by weight or less, 1,000 parts by weight or less, 500 parts by weight or less, 200 parts by weight or less, 175 parts by weight or less, 150 parts by weight or less, 125 parts by weight or less, 100 parts by weight or less, 80 parts by weight or less, 60 parts by weight or less, 40 parts by weight or less, 20 parts by weight or less, or 10 parts by weight or less relative to 1 part by weight of the fluorine-containing compound.

The amount of water may be 1 part by weight or more, 3 parts by weight or more, 5 parts by weight or more, 10 parts by weight or more, 20 parts by weight or more, 30 parts by weight or more, 40 parts by weight or more, 50 parts by weight or more, 100 parts by weight or more, 200 parts by weight or more, 300 parts by weight or more, 500 parts by weight or more, or 1,000 parts by weight or more, and 3,000 parts by weight or less, 2,000 parts by weight or less, 1,000 parts by weight or less, 500 parts by weight or less, 200 parts by weight or less, 175 parts by weight or less, 150 parts by weight or less, 125 parts by weight or less, 100 parts by weight or less, 80 parts by weight or less, 60 parts by weight or less, 40 parts by weight or less, 20 parts by weight or less, or 10 parts by weight or less relative to 1 part by weight of the fluorine-containing compound.

The amount of the organic solvent may be 1 part by weight or more, 3 parts by weight or more, 5 parts by weight or more, 10 parts by weight or more, 20 parts by weight or more, 30 parts by weight or more, 40 parts by weight or more, 50 parts by weight or more, 100 parts by weight or more, 200 parts by weight or more, 300 parts by weight or more, 500 parts by weight or more, or 1,000 parts by weight or more, and 3,000 parts by weight or less, 2,000 parts by weight or less, 1,000 parts by weight or less, 500 parts by weight or less, 200 parts by weight or less, 175 parts by weight or less, 150 parts by weight or less, 125 parts by weight or less, 100 parts by weight or less, 80 parts by weight or less, 60 parts by weight or less, 40 parts by weight or less, 20 parts by weight or less, or 10 parts by weight or less relative to 1 part by weight of the fluorine-containing compound.

### {Silicone}

The water- and oil-repellent agent in the present disclosure may include silicone (polyorganosiloxane). Containing the silicone enables providing favorable texture and durability in addition to favorable liquid-repellency.

As the silicone, a known silicone can be used, and examples of the silicone include a polydimethylsiloxane and modified silicones (for example, amino-modified silicone, epoxy-modified silicone, carboxy-modified silicone, and methylhydrogen silicone). For example, the silicone may be silicone wax having waxy properties. These may be used singly or in combination of two or more thereof.

A weight-average molecular weight of the silicone may be 1,000 or more, 10,000 or more, or 50,000 or more, and may be 500,000 or less, 250,000 or less, 100,000 or less, or 50,000 or less.

### [Amount of Silicone]

The amount of silicone is 0.1 parts by weight or more, 1 part by weight or more, 3 parts by weight or more, 5 parts by weight or more, 10 parts by weight or more, 15 parts by weight or more, 20 parts by weight or more, 50 parts by weight or more, 75 parts by weight or more, or 100 parts by weight or more, relative to 100 parts by weight of the fluorine-containing compound, and may be 500 parts by weight or less, 300 parts by weight or less, 200 parts by weight or less, 100 parts by weight or less, 50 parts by weight or less, 40 parts by weight or less, 30 parts by weight or less, 20 parts by weight or less, 10 parts by weight or less, or 5 parts by weight or less.

### {Wax}

The water- and oil-repellent agent in the present disclosure may include wax. Containing the wax can impart favorable liquid-repellency to a substrate.

Examples of wax include paraffin wax, microcrystalline wax, Fischer-Tropsch wax, polyolefin wax (for example, polyethylene wax and polypropylene wax), oxidized polyolefin wax, silicone wax, animal and vegetable wax and mineral wax. Paraffin wax is preferred. Specific examples of compounds constituting wax include n-alkane (such as tricosane, tetracosane, pentacosane, hexacosane, heptacosan, octacosan, nonacosan, triacontane, hentriacontane, dotriacontane, tritriacontane, tetratriacontane, pentatriacontane, hexatriacontane), n-alkene (such as 1-icosene, 1-docosene, 1-tricosene, 1-tetracosene, 1-pentacosene, 1-hexacosene, 1-heptacosene, 1-octacosene, 1-nonacosene, 1-triacontane, 1-hentriacontane, 1-dotriacontane, 1-tritriacontane, 1-tetratriacontane, 1-pentatriacontane, 1-hexatriacontane). The number of carbon atom in the compound constituting the wax is preferably 20 to 60, for example 25 to 45. A molecular weight of the wax may be 200 to 2,000, for example, 250 to 1,500 or 300 to 1,000. These may be used singly or in combination of two or more thereof.

The wax may have a melting point of 40°C or higher, 50°C or higher, 55°C or higher, 60°C or higher, 65°C or higher, or 70°C or higher, preferably 55°C or higher, more preferably 60°C or higher, and 200°C or lower, 150°C or lower, 130°C or lower, 120°C or lower, 110°C or lower, 100°C or lower, 80°C or lower, or 50°C or lower, and preferably 120°C or lower. The melting point of wax is measured according to JIS K 2235-1991.

### [Amount of Wax]

The amount of wax may be 0.1 parts by weight or more, 1 part by weight or more, 3 parts by weight or more, 5 parts by weight or more, 10 parts by weight or more, 15 parts by weight or more, 20 parts by weight or more, 50 parts by weight or more, 75 parts by weight or more, or 100 parts by weight or more, relative to 100 parts by weight of the fluorine-containing compound, and may be 500 parts by weight or less, 300 parts by weight or less, 200 parts by weight or less, 100 parts by weight or less, 50 parts by weight or less, 40 parts by weight or less, 30 parts by weight or less, 20 parts by weight or less, 10 parts by weight or less, or 5 parts by weight or less.

### {Organic Acid}

The water- and oil-repellent agent of the present disclosure may contain an organic acid. As the organic acid, a known organic acid can be used. Examples of the organic acid preferably include, for example, a carboxylic acid, a sulfonic acid, and a sulfinic acid, with the carboxylic acid being particularly preferred. Examples of the carboxylic acid include, for example, formic acid, acetic acid, propionic acid, butyric acid, oxalic acid, succinic acid, glutaric acid, adipic acid, malic acid, and citric acid, with the formic acid or acetic acid being particularly preferred. In the present disclosure, one type of organic acid may be used, or two or more thereof may be combined for use. For example, formic acid and acetic acid may be combined for use.

### [Amount of Organic Acid]

The amount of organic acid may be 0.1 parts by weight or more, 1 part by weight or more, 3 parts by weight or more, 5 parts by weight or more, 10 parts by weight or more, 15 parts by weight or more, 20 parts by weight or more, 50 parts by weight or more, 75 parts by weight or more, or 100 parts by weight or more, relative to 100 parts by weight of the fluorine-containing compound, and may be 500 parts by weight or less, 300 parts by weight or less, 200 parts by weight or less, 100 parts by weight or less, 50 parts by weight or less, 40 parts by weight or less, 30 parts by weight or less, 20 parts by weight or less, 10 parts by weight or less, or 5 parts by weight or less. The amount of organic acid may be adjusted so that a pH of the water- and oil-repellent agent is 3 to 10, for example 5 to 9, particularly 6 to 8. For example, the water- and oil-repellent agent may be acidic (pH of 7 or less, for example 6 or less).

### {Curing Agent}

The water- and oil-repellent agent of the present disclosure may contain a curing agent (active hydrogen-reactive compound or active hydrogen-containing compound).

The curing agent (cross-linking agent) in the water- and oil-repellent agent can effectively cure the fluorine-containing compound. The curing agent may be an active hydrogen-reactive compound or an active hydrogen-containing compound, which reacts with an active hydrogen or an active hydrogen-reactive group that the fluorine-containing compound has. Examples of the active hydrogen-reactive compound include an isocyanate compound, epoxy compound, chloromethyl group-containing compound, carboxyl group-containing compound, and hydrazide compound. Examples of the active hydrogen-containing compound include a hydroxyl group-containing compound, an amino group-containing compound and a carboxyl group-containing compound, a ketone group-containing compound, a hydrazide compound, and a melamine compound.

The curing agent may contain an isocyanate compound. The isocyanate compound may be a polyisocyanate compound. The polyisocyanate compound is a compound having two or more isocyanate groups in one molecule. The polyisocyanate compound serves as a cross-linking agent. Examples of the polyisocyanate compound include, for example, an aliphatic polyisocyanate, an alicyclic polyisocyanate, an araliphatic polyisocyanate, an aromatic polyisocyanate, and derivatives of these polyisocyanates. The isocyanate compound may be a blocked isocyanate compound (for example, a blocked polyisocyanate compound). The blocked isocyanate compound is a compound in which an isocyanate group of an isocyanate compound is masked with a blocking agent to inhibit reaction.

Examples of the aliphatic polyisocyanates are aliphatic triisocyanates such as trimethylene diisocyanate, tetramethylene diisocyanate, hexamethylene diisocyanate, pentamethylene diisocyanate, 1,2-propylene diisocyanate, 1,2-butylene diisocyanate, 2,3-butylene diisocyanate, 1,3-butylene diisocyanate, 2,4,4- or 2,2,4-trimethylhexamethylene diisocyanate, an aliphatic diisocyanate of 2,6-diisocyanatomethylcaproate, and aliphatic triisocyanates such as lysine ester triisocyanate, 1,4,8-triisocyanateoctane, 1,6,11-triisocyanatoundecane, 1,8-diisocyanato-4-isocyanatomethyloctane, 1,3,6-triisocyanatohexane, 2,5,7-trimethyl-1,8-diisocyanato-5-isocyanatomethyloctane. These may be used singly or in combination of two or more thereof.

Examples of the alicyclic polyisocyanates include, for example, an alicyclic diisocyanate and an alicyclic triisocyanate. Specific examples of the alicyclic polyisocyanate include 1,3-cyclopentene diisocyanate, 3-isocyanatomethyl-3,5,5-trimethylcyclohexyl isocyanate (isophorone diisocyanate), and 1,3,5-triisocyanatocyclohexane. These may be used singly or in combination of two or more thereof.

Examples of the aromatic-aliphatic polyisocyanate include an aromatic-aliphatic diisocyanate and aromatic-aliphatic triisocyanate. Specific examples of the araliphatic polyisocyanate include 1,3- or 1,4-xylylene diisocyanate or a mixture thereof, 1,3- or 1,4-bis(1-isocyanato-1-methylethyl)benzene (tetramethyl xylylene diisocyanate) or a mixture thereof, and 1,3,5-triisocyanatomethylbenzene. These may be used singly or in combination of two or more thereof.

Examples of the aromatic polyisocyanates include an aromatic diisocyanate, aromatic triisocyanate, and aromatic tetraisocyanate. Specific examples of the aromatic polyisocyanate include, for example, m-phenylene diisocyanate, p-phenylene diisocyanate, 4,4'-diphenyl diisocyanate, 1,5-naphthalene diisocyanate, 2,4'- or 4,4'-diphenylmethane diisocyanate, or a mixture thereof, 2,4- or 2,6-tolylene diisocyanate or a mixture thereof, triphenylmethane-4,4',4"-triisocyanate, and **4,4'-**diphenylmethane-2,2',5,5'-tetraisocyanate. These may be used singly or in combination of two or more thereof.

Examples of the derivative of the polyisocyanate include various derivatives such as a dimer, trimer, biuret, allophanate, carbodiimide, urethodione, urethoimine, isocyanurate, and iminooxadiazinedione of the aforementioned polyisocyanate compounds. These may be used singly or in combination of two or more thereof.

These polyisocyanates can be used singly or in combination of two or more thereof.

As the polyisocyanate compound, a blocked polyisocyanate compound (blocked isocyanate), which is a compound obtained by blocking isocyanate groups of the polyisocyanate compound with a blocking agent, is preferably used. The blocked polyisocyanate compound is preferably used because it is relatively stable even in solution and can be used in the same solution as solution of the water- and oil-repellent agent.

The blocking agent is an agent that blocks free isocyanate groups. The blocked polyisocyanate compound, for example, can be heated 100°C or higher, for example, 130°C or higher to regenerate isocyanate groups, facilitating a reaction with hydroxyl groups. Examples of the blocking agent include, for example, a phenolic compound, lactam-based compound, aliphatic alcohol-based compound, and oxime-based compound. The polyisocyanate compound may be used singly or in combination of two or more thereof.

The epoxy compound is a compound having an epoxy group. Examples of the epoxy compound include epoxy compounds having a polyoxyalkylene group, such as a polyglycerol polyglycidyl ether and a polypropylene glycol diglycidyl ether; as well as a sorbitol polyglycidyl ether.

The chloromethyl group-containing compound is a compound having a chloromethyl group. Examples of the chloromethyl group-containing compound include, for example, a chloromethyl polystyrene.

The carboxyl group-containing compound is a compound having a carboxyl group. Examples of the carboxyl group-containing compound include, for example, a (poly)acrylic acid, and a (poly)methacrylic acid.

Specific examples of the ketone group-containing compound include, for example, a (poly)diacetone acrylamide, and diacetone alcohol.

Specific examples of the hydrazide compound include, for example, hydrazine, a carbohydrazide, and adipic acid hydrazide.

Specific examples of the melamine compound include, for example, a melamine resin and a methyl etherified melamine resin.

### [Amount of Curing Agent]

The amount of the curing agent may be 0.1 parts by weight or more, 1 part by weight or more, 3 parts by weight or more, 5 parts by weight or more, 10 parts by weight or more, 15 parts by weight or more, 20 parts by weight or more, 50 parts by weight or more, 75 parts by weight or more, or 100 parts by weight or more, relative to 100 parts by weight of the fluorine-containing compound, and may be 500 parts by weight or less, 300 parts by weight or less, 200 parts by weight or less, 100 parts by weight or less, 50 parts by weight or less, 40 parts by weight or less, 30 parts by weight or less, 20 parts by weight or less, 10 parts by weight or less, and 5 parts by weight or less.

### {Other Component}

The water- and oil-repellent agent may contain a component other than the aforementioned components. Examples of the other components include, for example, polysaccharides, a paper strengthening agent, an agglomerating agent, a yield improver, a coagulant, a binder resin, an anti-slip agent, a sizing agent, a paper strengthening agent, PVA, a penetrant, an organic acid, a pigment, a filler, an antistatic agent, an antiseptic agent, an ultraviolet absorber, an antibacterial agent, a deodorant, and a fragrance. These may be used singly or in combination of two or more thereof.

In addition to the above components, as other components, for example, other water-repellent and/or oil-repellent agents, a dispersant, a texture modifier, a softening agent, a flame retarder, a coating material fixing agent, a wrinkle-resistant agent, a drying rate adjuster, a cross-linking agent, a film formation agent, a compatibilizer, an antifreezing agent, a viscosity adjuster, an ultraviolet absorber, an antioxidant, a pH adjuster, an insect repellent, an antifoaming agent, an anti-shrinkage agent, a laundry wrinkle-resistant agent, a shape retention agent, a drape retention agent, an ironing improving agent, a brightening agent, a whitening agent, fabric softening clay, a migration-proofing agent such as a polyvinylpyrrolidone, a polymer dispersant, a soil release agent, a scum dispersant, a fluorescent brightening agent such as 4,4-bis(2-sulfostyryl)biphenyldisodium (Tinopal CBS-X manufactured by Ciba Specialty Chemicals Plc), a dye fixing agent, an anti-color fading agent such as 1,4-bis(3-aminopropyl)piperazine, a stain removing agent, enzymes such as cellulase, amylase, protease, lipase, and keratinase as fiber surface modifiers, a foam inhibitor, and silk protein powder that can impart texture and functions of silk such as moisture absorption and release properties, and surface modified products or emulsified dispersions thereof (for example, K-50, K-30, K-10, A-705, S-702, L-710, FP series (Idemitsu Petrochemical Co., Ltd.), hydrolyzed silk liquid (Jomo), SILKGEN G Soluble S (ICHIMARU PHARCOS Co., Ltd.)), an antifouling agent (for example, a nonionic polymer compound composed of an alkylene terephthalate and/or an alkylene isophthalate units and a polyoxyalkylene unit (for example, FR627 manufactured by GOO CHEMICAL CO., LTD.), SRC-1 manufactured by Clariant (Japan), K. K.), can be compounded. These may be used singly or in combination of two or more thereof.

### [Polysaccharide]

Examples of polysaccharides include starch, xanthan gum, karaya gum, welan gum, guar gum, pectin, tamarind gum, carrageenan, chitosan, gum arabic, locust bean gum, cellulose, alginic acid, agar, dextran, cellulose, carboxymethylcellulose, hydroxyethylcellulose, hydroxypropylcellulose, chitin nanofiber, cellulose nanofiber and pullulan. The polysaccharide may be a substituted modified polysaccharide, particularly a modified polysaccharide having a hydroxyl group or a cationic group introduced therein.

### [Paper Strengthening Improver, Agglomerating agent, Yield Improver or Coagulant]

Examples of the paper strengthening improver, agglomerating agent, yield improver or coagulant include, for example, a styrenic polymer (styrene/maleic acid polymer, styrene/acrylic acid polymer), a urea-formaldehyde polymer, a polyethyleneimine, a melamine-formaldehyde polymer, a polyamidoamine-epichlorohydrin polymer, a polyacrylamide-based polymer, a polyamine-based polymer, a polydiallyldimethylammonium chloride, a alkylamine-epichlorohydrin condensate, a condensate of alkylene dichloride and polyalkylenepolyamine, a dicyandiamide formalin condensate, a dimethyldiallylammonium chloride polymer, and an olefin/maleic anhydride polymer.

### [Sizing Agent]

Examples of the sizing agent include a cellulose-reactive sizing agent, for example, a rosin-based sizing agent such as rosin-based soap, rosin-based emulsion/a dispersion, a cellulose-reactive sizing agent, for example, emulsion/dispersions of acid anhydrides such as alkyl and alkenyl succinic anhydrides (ASA), an alkenyl and alkyl ketene dimers (AKD) and multimers thereof, and anionic, cationic and amphoteric polymers of ethylenically unsaturated monomers, for example, a styrene and acrylate copolymer.

### [Antistatic Agent]

Examples of the antistatic agent include, for example, cationic antistatic agents having cationic functional groups such as a quaternary ammonium salt, a pyridinium salt, and primary, secondary, and tertiary amino groups; anionic antistatic agents having anionic functional groups such as a sulfonate salt and a sulfate ester salt, a phosphonate and a phosphate ester salt; amphoteric antistatic agents such as an alkyl betaine and a derivative thereof, imidazoline and a derivative thereof, and alanine and a derivative thereof; and nonionic antistatic agents such an amino alcohol and a derivative thereof, glycerin and a derivative thereof, and a polyethylene glycol and a derivative thereof. For example, an ion conductive polymer obtained by polymerizing or copolymerizing a monomer having an ion conductive group of the cationic, anionic, or amphoteric antistatic agent, may be used. These may be used singly or in combination of two or more thereof.

### [Antiseptic Agent]

The antiseptic agent may be used mainly to enhance antisepsis power and bactericidal power to maintain antiseptic during long-term storage. Examples of the antiseptic agent include isothiazolone-based organosulfur compounds, benzisothiazolone-based organosulfur compounds, benzoic acids, and 2-bromo-2-nitro-1,3-propanediol.

### [Ultraviolet Absorber]

The ultraviolet absorber is an agent that has a protection effect against ultraviolet rays, and is a component that absorbs ultraviolet rays, converts them into infrared rays, visible rays, and the like, and emits them. Examples of the ultraviolet absorber include aminobenzoic acid derivatives, salicylic acid derivatives, silicic acid derivatives, benzophenone derivatives, azole-based compounds, and 4-t-butyl-4'-methoxybenzoylmethane.

### [Antibacterial Agent]

The antibacterial agent is a component that exhibits the effect of inhibiting bacteria from growing on fibers and further exhibits the effect of inhibiting generation of unpleasant odors derived from decomposition products of microorganisms. Examples of the antibacterial agents include, for example, cationic antibacterial agents such as a quaternary ammonium salt, bis-(2-pyridylthio-1-oxide) zinc, a polyhexamethylene biguanidine hydrochloride salt, 8-oxyquinoline, and a polylysine.

### [Deodorant]

Examples of the deodorant include cluster dextrin, methyl-β-cyclodextrin, 2-hydroxypropyl-β-cyclodextrin, monoacetyl-β-cyclodextrin, acylamidopropyl dimethylamine oxide, and an aminocarboxylic acid-based metal complex (the zinc complex of trisodium methylglycine diacetate described in WO2012/090580).

### [Amount of Other Component]

Each amount or the total amount of other components may be 0.1 parts by weight or more, 1 part by weight or more, 3 parts by weight or more, 5 parts by weight or more, 10 parts by weight or more, 15 parts by weight or more, 20 parts by weight or more, 50 parts by weight or more, 75 parts by weight or more, or 100 parts by weight or more, relative to 100 parts by weight of the fluorine-containing compound, and may be 500 parts by weight or less, 300 parts by weight or less, 200 parts by weight or less, 100 parts by weight or less, 50 parts by weight or less, 40 parts by weight or less, 30 parts by weight or less, 20 parts by weight or less, 10 parts by weight or less, or 5 parts by weight or less.

### <Production Method of Treated Textile Product or Paper Product>

A method for producing a product treated with the water- and oil-repellent agent in the present disclosure includes a treatment step of treating a substrate with the water- and oil-repellent agent described above.

The "treatment" refers to applying the water- and oil-repellent agent to a substrate by dipping, spraying, coating, etc. By the treatment, a fluorine-containing compound that is an active ingredient of the water- and oil-repellent agent, adheres to an inside and/or on a surface of the substrate. Herein, the adhesion may be physical adhesion or chemical adhesion, and, for example, the fluorine-containing compound may be physically or chemically (by reaction) modified onto a hydroxyl group of a substrate (for example, a fiber, paper, and glass).

### [Substrate]

Substrates to be treated with the water- and oil-repellent agent in the present disclosure are not limited, and are preferably a textile product or a paper product, and in particular, a paper product.

The water- and oil-repellent agent in the present disclosure imparts liquid-repellency to a substrate (e.g., fiber substrate, paper substrate), and may function as at least one selected from a water-repellent agent, an oil-repellent agent, an oil-resistant agent and a water-resistant agent. The substrate which has been treated with the water- and oil-repellent agent in the present disclosure, for example, is an oil-repellent paper or a water-repellent paper.

Examples of the substrate for textile products include animal and vegetable natural fibers such as cotton, linen, wool, and silk, synthetic fibers such as a polyamide, a polyester, a polyvinyl alcohol, a polyacrylonitrile, a polyvinyl chloride, and a polypropylene, and semi-synthetic fibers such as rayon and acetate, inorganic fibers such as a glass fiber, a carbon fiber, and an asbestos fiber, or blended fibers thereof. The textile products include a woven fabric, a knitted fabric, a nonwoven fabric, fabric in the form of clothing (for example, water-repellent garments, for example, a raincoat) and carpets, and a fiber, yarn and intermediate fiber product (for example, a sliver or a crude yarn) in a state of before being formed into fabric, may undergo treatment.

Examples of the substrate for paper products include, for example, bleached or unbleached chemical pulp such as kraft pulp or sulfite pulp, bleached or unbleached high-yield pulp such as groundwood pulp, mechanical pulp, or thermomechanical pulp, paper made from wastepaper pulp such as wastepaper of newspapers, magazines, and cardboard, and deinked wastepaper, a container made of paper, and a molded product made of paper. Specific examples of the paper products include, for example, a food packaging material, a food container, gypsum liner board base paper, coated base paper, medium-quality paper, a general liner and core, neutral pure white roll paper, a neutral liner, a rust-proof liner, and metal pasted paper, kraft paper, neutral printing writing paper, neutral coated base paper, neutral PPC paper, neutral thermal paper, neutral pressure-sensitive base paper, neutral inkjet paper and neutral information paper, and molded paper (mold container), and suitable examples thereof include the food packaging material and the food container.

A substrate to be treated with the water- and oil-repellent agent of the present disclosure is not limited to textile products or paper products, and it may also include, for example, stone, a filter (for example, an electrostatic filter), a dust-protective mask, fuel cell parts (for example, a gas diffusion electrode and a gas diffusion support), glass, wood, leather, fur, asbestos, brick, cement, metal and oxide, a ceramic product, a plastic, a painted surface, and a plaster.

When the substrate is glass, a glass product to be produced may be an optical member. A certain layer (or film), such as a hard coat layer or an antireflection layer, may be formed on the surface (outermost layer) of a glass substrate. The antireflection layer may be any of a single-layer antireflection layer and a multi-layer antireflection layer. Examples of inorganic substances usable in the antireflection layer include SiO₂, SiO, ZrO₂, TiO₂, TiO, Ti₂O₃, Ti₂O₅, Al₂O₃, Ta₂O₅, CeO₂, MgO, Y₂O₃, SnO₂, MgF₂, and WO₃. One of these inorganic substances may be used singly, or two or more may be used in combination (e.g., as a mixture). In the case of a multi-layer antireflection layer, SiO₂ and/or SiO is preferably used in the outermost layer thereof. When the article to be produced is an optical glass component for a touch panel, a part of the surface of the substrate (glass) may have a transparent electrode such as a thin film in which indium tin oxide (ITO), indium zinc oxide, or the like is used. The substrate, according to its specific configuration or the like, may have an insulating layer, an adhesive layer, a protecting layer, a decorated frame layer (I-CON), an atomizing film layer, a hard coating layer, a polarizing film, a phase difference film, a liquid crystal display module, or the like.

### [Treatment Method]

The water- and oil-repellent agent of the present disclosure can be applied to a substrate as a treatment agent (particularly a surface-treating agent) by a conventionally known method. The treatment method may be a method for dispersing the water- and oil-repellent agent in the present disclosure in an organic solvent or water, if necessary, to dilute it and allowing it to adhere to an inside of a substrate and/or on a surface thereof by a known method such as dip coating, spray coating, and foam coating. After drying, a product to which a solid component of the water- and oil-repellent agent has been adhered, is obtained. If necessary, the water and oil-repellent agent of the present disclosure may be applied in combination of a suitable cross-linking agent, and curing may be carried out. If necessary, the water- and oil-repellent agent of the present disclosure can be further combined for use with various additives such as water- and/or oil-repellent agents, an anti-slip agent, an antistatic agent, a texture modifier, a softening agent, an antibacterial agent, a flame retarder, a coating material fixing agent, a wrinkle-resistant agent, a drying rate adjuster, a cross-linking agent, a film formation agent, a compatibilizer, an antifreezing agent, a viscosity modifier, an ultraviolet absorber, an antioxidant, a pH adjuster, an insect repellent, and an antifoaming agent. Examples of the various additives may be the same as those explained in the section "Other Component" described above. A concentration of the water- and oil-repellent agent in a treatment agent brought into contact with a substrate may be appropriately changed depending on its use, and may be 0.01 to 10% by weight, for example 0.05 to 5% by weight.

The water- and oil-repellent agent can be applied to a substrate by any of methods known for treating a substrate with liquid. The substrate may be immersed in the water- and oil-repellent agent, or solution may be adhered or sprayed onto the substrate. The treated substrate is preferably dried and cured by heating in order to develop liquid-repellency. The heating temperature may be, for example, 100°C to 200°C, 100°C to 170°C, or 100°C to 120°C. Favorable performance can be obtained even by heating at lowered temperatures (for example, 100°C to 140°C) in the present disclosure. In the present disclosure, the heating time may be 5 seconds to 60 minutes, for example, 30 seconds to 3 minutes. When the substrate is paper, the paper may undergo coating, or solution may be adhered or sprayed onto the paper, or the solution may be mixed with pulp slurry before papermaking to undergo treatment. The treatment may be external addition treatment or internal addition treatment. Alternatively, the water- and oil-repellent agent may be applied to a textile product by a cleaning method, and for example, it may be applied to a textile product in, for example, washing application or a dry cleaning method.

### [Paper product Treatment]

Examples of paper substrates include paper, a container made of paper, and a molded product made of paper (for example, a pulp mold). The fluorine-containing compound of the present disclosure adheres well to the paper substrate.

The paper can be produced by a conventionally known papermaking method. An internal addition treatment method in which the water- and oil-repellent agent is added to pulp slurry before papermaking, or an external addition treatment method in which the water- and oil-repellent agent is applied to paper after papermaking, can be employed.

A size press used in an external addition treatment method, can be divided into the following types depending on a coating method.

One coating method involves supplying a coating liquid (size liquid) to a nip portion formed by passing paper between two rubber rolls, creating a pool of the coating liquid called a pond, and allowing the paper to pass through this pool to coat both sides of the paper with the size liquid, which is a method employed for a so-called pound-type two-roll size press. Another coating method is a method used for a gate roll type size press in which a size liquid is applied by a surface transfer type, and a rod metering type size press. In the pound-type two-roll size press, the size liquid easily penetrates into an inside of paper, and in the surface transfer type, a size liquid component is likely to stay on a surface of the paper. In the surface transfer type, a coating layer is likely to stay on a surface of paper more than in the pound-type two-roll size press, and the amount of coating layer formed on the surface is more than in the pound-type two-roll size press. In the present disclosure, even in the case of using the former pound-type two-roll size press, performance can be imparted to paper. After having been lightly dried at room temperature or elevated temperature, the paper treated in such a manner is arbitrarily accompanied by heat treatment that can have a temperature range of up to 300°C, for example up to 200°C, and particularly the temperature range of 80°C to 180°C, depending on the nature of the paper, as a result of which excellent oil resistance, water resistance, and the like can be exhibited.

The internal addition treatment method may refer to a treatment method for adding the water- and oil-repellent agent to pulp slurry before papermaking. The internal addition treatment method may include, but is not limited to, one or more of a step of adding the water- or oil-repellent agent to pulp slurry and stirring and mixing them; a step of sucking and dehydrating the pulp composition prepared in the step through a net-like body of predetermined shape and depositing the pulp composition then to form a pulp mold intermediate; and a step of molding and drying the pulp mold intermediate by using a heated molding mold to obtain paper, a container made of paper, and a molded product made of paper. After having been lightly dried at room temperature or elevated temperature, the treated paper may be arbitrarily subjected to heat treatment, depending on the nature of the paper. Temperature of the heat treatment may be 150°C or higher, 180°C or higher, or 210°C or higher, and may also be 300°C or lower, 250°C or lower, or 200°C or lower, particularly 80°C to 180°C. Carrying out the heat treatment in such a temperature range enables exhibiting, for example, excellent oil resistance and water resistance.

The present disclosure can be used, for example, in gypsum liner board base paper, coated base paper, medium-quality paper, a general liner and core, neutral pure white roll paper, a neutral liner, a rust-proof liner, and metal pasted paper, and kraft paper. The present disclosure can also be used, for example, in neutral printing writing paper, neutral coated base paper, neutral PPC paper, neutral thermal paper, neutral pressure-sensitive base paper, neutral inkjet paper and neutral information paper.

As pulp raw materials, any of bleached or unbleached chemical pulp such as kraft pulp or sulfite pulp; bleached or unbleached high-yield pulp such as groundwood pulp, mechanical pulp, or thermomechanical pulp; and wastepaper pulp such as wastepaper of newspapers, magazines, and cardboard, or deinked wastepaper, can be used. Moreover, a blend of the aforementioned pulp raw material and a synthetic fibers such as asbestos, a polyamide, a polyimide, a polyester, a polyolefin, or a polyvinyl alcohol, can also be used.

A sizing agent can be added to improve water resistance of paper. Examples of the sizing agent are a cationic sizing agent, an anionic sizing agent, and a rosin-based sizing agent (for example, an acidic rosin-based sizing agent and a neutral rosin-based sizing agent) . The amount of sizing agent may be 0.01 to 5% by weight relative to pulp.

For paper, as chemicals for papermaking used to such an extent that they are usually used, if necessary, paper strengthening agents such as starch, modified starch, carboxymethyl cellulose, and a polyamide polyamine-epichlorohydrin resin, and additives used in production of paper, such as an agglomerating agent, a fixing agent, a yield improver, a dye, a fluorescent dye, a slime control agent, and an antifoaming agent, can be used. Starch and modified starch are preferably used. If necessary, paper can be coated with the water- and oil-repellent agent together with starch, a polyvinyl alcohol, a dye, a coating color, an anti-slip agent, or the like, for example, by a size press, a gate roll coater, a bill blade coater, a calender, or the like.

In external addition, the amount of fluorine-containing compound contained in a coating layer is preferably 0.01 to 2.0 g/m² and particularly 0.1 to 1.0 g/m². The coating layer is preferably formed of the water- and oil-repellent agent and starch and/or modified starch. The solid content of the water- and oil-repellent agent for paper in the coating layer is preferably 2 g/m² or less.

In internal addition, the water- and oil-repellent agent is preferably mixed with pulp so that the amount of water- and oil-repellent agent is 0.01 to 50 parts by weight or 0.01 to 30 parts by weight, for example 0.01 to 10 parts by weight and particularly 0.2 to 5.0 parts by weight, relative to 100 parts by weight of pulp that forms paper.

In external addition, even when employing a so-called pound type two-roll size press treatment in which a treatment liquid is stored between rolls, and base paper is passed through the treatment liquid between the rolls at an arbitrary roll speed and nip pressure, oil-repellency can be imparted to paper.

In external addition treatment, a paper substrate may contain additives such as a sizing agent, a paper strengthening agent, an agglomerating agent, or yield improver or a coagulant. The additives may be nonionic, cationic, anionic or amphoteric. An ionic charge density of the additive may be -10,000 to 10,000 µeq/g, preferably -4,000 to 8,000 µeq/g, and more preferably -1,000 to 7,000 µeq/g. The additive (solid content or active ingredient) such as a sizing agent, a paper strengthening agent, an agglomerating agent, a yield improver, or a coagulant may be generally used in an amount of 0.1 to 10% by weight (for example, 0.2 to 5.0% by weight) relative to pulp. In the case of a paper substrate containing a cationic additive (for example, a sizing agent, a paper strengthening agent, an agglomerating agent, a yield improver, or a coagulant), the water- and oil-repellent agent may be anionic, and in the case of a paper substrate containing an anionic additive, the water- and oil-repellent agent may be cationic, but paper substrates and additives are not limited thereto.

In internal addition treatment, pulp slurry having a pulp concentration of 0.5 to 5.0% by weight (for example, 2.5 to 4.0% by weight) preferably undergo papermaking. The pulp slurry can be added with an additive (for example, a sizing agent, a paper strengthening agent, an agglomerating agent, a yield improver, or a coagulant) and the fluorine-containing compound. Examples of the additive (for example, a sizing agent, a paper strengthening agent, an agglomerating agent, a yield improver, or a coagulant) include an alkyl ketene dimer, an alkenyl succinic anhydride, a styrenic polymer (styrene/maleic acid polymer, styrene/acrylic acid-based polymer), a urea-formaldehyde polymer, a polyethyleneimine, a melamine-formaldehyde polymer, a polyamideamine-epichlorohydrin polymer, a polyacrylamide-based polymer, a polyamine-based polymer, a polydiallyldimethylammonium chloride, an alkylamine-epichlorohydrin condensate, a condensate of alkylene dichloride and polyalkylene polyamine, a dicyandiamide·formalin condensate, a dimethyldiallylammonium chloride polymer, and an olefin/maleic anhydride polymer.

In the internal addition treatment, the paper substrate may contain an additive such as a sizing agent, a paper strengthening agent, an agglomerating agent, a yield improver or a coagulant. The additive may be nonionic, cationic, anionic or amphoteric. The additive may have an ionic charge density of -10,000 to 10,000 µeq/g, preferably -4,000 to 8,000 µeq/g, and more preferably - 1,000 to 7,000 µeq/g. The additive (solid content or active ingredient) such as a sizing agent, a paper strengthening agent, an agglomerating agent, a yield improver, or a coagulant may be generally used in an amount of 0.1 to 10% by weight (for example, 0.2 to 5.0% by weight) relative to pulp. In the case of a paper substrate containing a cationic additive (for example, a sizing agent, a paper strengthening agent, an agglomerating agent, a yield improver, or a coagulant), the water- and oil-repellent agent may be anionic, and in the case of a paper substrate containing an anionic additive, the water- and oil-repellent agent may be cationic, but paper substrates and additives are not limited thereto.

### [Pretreatment of Fiber Product]

The fiber product may be pretreated before being treated with the water- and oil-repellent agent of the present disclosure. Pretreatment of the fiber product enables imparting excellent fastness to a fiber product after treated with the water- and oil-repellent agent.

Examples of the pretreatment of fiber product include, for example, cationization treatment such as reaction with a reactive quaternary ammonium salt, anionization treatment such as sulfonation, carboxylation, and phosphorylation, acetylation treatment after the anionization treatment, benzoylation treatment, carboxymethylation treatment, graft treatment, tannic acid treatment, and polymer coating treatment.

A method for pretreating the fiber product is not limited, but the fiber product can be pretreated by a conventionally known method. A method for dispersing a pretreatment liquid in an organic solvent or water, if necessary, to dilute the pretreatment liquid and adhering it to an inside of the fiber product and/or on a surface thereof and drying the liquid by a known method such as dip coating, spray coating, or foam coating, may be employed. The pH, temperature, etc. of the pretreatment liquid may be adjusted according to an extent of treatment desired. As an example of the method for pretreating a fiber product, a method for pretreating a fiber product with the treatment agent described above will be described in detail.

The pretreatment method of a fiber product may involve a step of imparting a fiber with one or more functional groups (hereinafter sometimes referred to as "specific functional groups") selected from the group consisting of the monovalent group represented by -SO₃M¹ (wherein M¹ represents a monovalent cation) or the monovalent group represented by -COOM² (wherein M² represents a monovalent cation), and the monovalent group represented by **-**OP(O)(OX¹)(OX²) (wherein X¹ and X² each independently represent a hydrogen atom or an alkyl group having 1 to 22 carbon atoms).

Examples of M¹ include H, K, Na, or an ammonium ion which may have a substituent. Examples of M² include H, K, Na, or an ammonium ion which may have a substituent. When X¹ or X² is an alkyl group, it is preferably an alkyl group having 1 to 22 carbon atoms and more preferably an alkyl group having 4 to 12 carbon atoms.

A fiber containing the above specific functional group (hereinafter sometimes referred to as a "functional group-containing fiber") can be prepared, for example, by the following method.
(i) A compound having the above specific functional group is allowed to adhere to a fiber material. The adhesion of the compound may be in a condition such that a portion of the compound and a portion of the fibers are chemically bonded as long as the above specific functional groups remain in a sufficient amount.
(ii) A fiber is prepared by directly introducing the above specific functional group into the material constituting the fiber.

In the case of (i), for example, a functional group-containing fiber can be obtained by treating the fiber material with a pretreatment liquid containing one or more compounds having the above specific functional group, namely, by the step of introducing the functional group.

Materials used for the fiber material, are not limited, and examples thereof include natural fibers such as cotton, linen, silk, and wool, semi-synthetic fibers such as rayon and acetate, synthetic fibers such as a polyamide (nylon, etc.), a polyester, a polyurethane, and a polypropylene, composite fibers thereof, blended fibers, and the like. A form of the fiber material may be any form such as a fiber (tow, sliver, etc.), a yarn, a knitted fabric (including an interknitted fabric), a woven fabric (including an interwoven fabric), or a nonwoven fabric.

In the present embodiment, from the viewpoint of improving water-repellency of the obtained textile product, a fiber material containing a polyamide and a polyester as raw materials, is preferably used, and in particular, nylon such as nylon 6, or nylon 6,6, a polyester such as a polyethylene terephthalate (PET), a polytrimethyl terephthalate, or polylactic acid, and blended fibers containing these, are preferably used.

A Phenolic polymer can be used as the compound having -SO₃M¹ described above. Examples of such a phenolic polymer include that containing at least one of compounds represented by the following general formula: 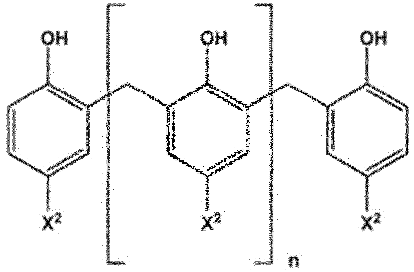 [wherein X² represents -SO₃M³, wherein M³ represents a monovalent cation, or a group represented by the following general formula, and n is an integer of 20 to 3,000.] 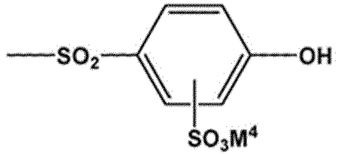 [wherein M⁴ represents a monovalent cation.].

Examples of M³ includes H, K, Na or an ammonium ion that may have a substituent.

Examples of M⁴ includes H, K, Na or an ammonium ion which may have a substituent.

The compounds represented by the general formula above may be, for example, formalin condensates of phenol sulfonic acid and formalin condensates of sulfonated bisphenol S.

Examples of the compound having -COOM² above include a polycarboxylic acid-based polymer.

As the polycarboxylic acid-based polymer, for example, a polymer synthesized by a conventionally known radical polymerization method using acrylic acid, methacrylic acid, maleic acid, or the like as a monomer, or a commercially available polymer, can be used.

A method for producing the polycarboxylic acid-based polymers may include, for example, adding a radical polymerization initiator to an aqueous solution of the aforementioned monomer and/or salt thereof and heating and reacting the mixture at 30 to 150°C for 2 to 5 hours. At this time, an aqueous solution of the above monomer and/or salt thereof may be added with aqueous solvents such as alcohols such as methanol, ethanol, and isopropyl alcohol, and acetone. Examples of the radical polymerization initiator include persulfates such as potassium persulfate, sodium persulfate, and ammonium persulfate, redox polymerization initiators in combination of the persulfate and sodium bisulfite or the like, hydrogen peroxide, and a water-soluble azo-based polymerization initiator. These radical polymerization initiators may be used singly or in combination of two or more thereof. Furthermore, a chain transfer agent (for example, octyl thioglycolate) may be added upon radical polymerization for the purpose of adjusting the degree of polymerization.

In addition to the aforementioned monomers, a copolymerizable monomer can be used for radical polymerization. Examples of the copolymerizable monomer include vinyl-based monomers such as ethylene, vinyl chloride, and vinyl acetate, an acrylamide, acrylates, and methacrylates. The acrylates and methacrylates preferably have a hydrocarbon group having 1 to 3 carbon atoms, which may have a substituent such as a hydroxyl group. Examples of such acrylates or methacrylates include methyl acrylate, methyl methacrylate, ethyl acrylate, ethyl methacrylate, 2-hydroxyethyl acrylate, 2-hydroxyethyl methacrylate, propyl acrylate, propyl methacrylate, and the like. These copolymerizable monomers may be used singly or in combination of two or more thereof.

A carboxyl group in the polycarboxylic acid-based polymer may be free or may be neutralized with an alkali metal, an amine-based compound, or the like. Examples of the alkali metal include sodium, potassium, lithium, and the like, and examples of the amine-based compound include ammonia, monoethanolamine, diethanolamine, triethanolamine, and the like.

The weight-average molecular weight of the polycarboxylic acid-based polymer is preferably 1,000 to 20,000 and more preferably from 3,000 to 15,000, from the viewpoint of favorable water-repellency of the resulting textile product.

As the polycarboxylic acid-based polymer, commercially available products such as "Neocrystal 770" (trade name, manufactured by NICCA CHEMICAL CO., LTD.) and "Ceropol PC-300" (trade name, manufactured by Sanyo Chemical Industries, Ltd.) can be used.

Examples of the compound having -O-P(O)(OX¹)(OX²) as described above include phosphoric acid ester compounds represented by the following general formula: 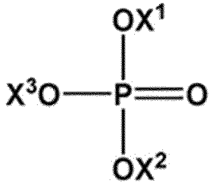 [wherein, X¹ or X² is the same as defined above, and X³ represents an alkyl group having 1 to 22 carbon atoms.]

As the aforementioned phosphoric acid ester compound, phosphoric acid monoesters, diesters and triesters, and mixtures thereof can be used in which the alkyl ester moiety is an alkyl group having 1 to 22 carbon atoms.

In view of favorable water-repellency of the textile products to be obtained, lauryl phosphoric acid ester and decyl phosphoric acid ester are preferably used.

As the phosphoric acid ester compound, for example, a commercially available product such as "Phosphanol ML-200" (trade name, manufactured by TOHO Chemical Industry Co., Ltd.) can be used.

A pretreatment liquid containing one or more of the compounds having the aforementioned specific functional group can be, for example, an aqueous solution of the compound described above. The pretreatment liquid may also contain an acid, alkali, surfactant, chelating agent, and the others.

Examples of the method for treating a fiber material with the above pretreatment liquid include padding treatment, dip treatment, spray treatment, and coating treatment. Examples of padding treatment include the method involving using the padding apparatus as described on pages 396 to 397 of Seni Sensyoku Kako Jiten (in Japanese; Fiber-dyeing process dictionary) (published by THE NIKKAN KOGYO SHIMBUN, LTD., 1963) and pages 256 to 260 of Irozome Kagaku (in Japanese; dyeing chemistry) III (published by Jikkyo Shuppan Co., Ltd., 1975). Examples of the coating treatment include the method involving using a coating machine as described on pages 473 to 477 of Sensyoku Shiage Kiki Soran (in Japanese; Comprehensive guide to dyeing and finishing machines) (published by Fiber Japan CO., LTD., 1981). Examples of the dip treatment include the method involving using a batch type dyeing machine as described in pages 196 to 247 of Sensyoku Shiage Kiki Soran (in Japanese) (published by Fiber Japan CO., LTD., 1981), and for example, a jet dyeing machine, air flow dyeing machine, drum dyeing machine, wince dyeing machine, washer dyeing machine, and cheese dyeing machine can be used. Examples of the spray treatment includes a method involving using an air spray that nebulizes and sprays a treatment liquid by compressed air, or an air spray by hydraulic pressure nebulization system. In this case, the concentration of the treatment liquid and treatment conditions of heat treatment after application can be adjusted appropriately, taking into consideration various conditions such as their purposes and performance. Moreover, in a case in which the pretreatment liquid contains water, it is preferably dried to remove water after the pretreatment liquid has been allowed to adhere to the fiber material. The drying method are not limited, and either a dry heat method or a wet heat method may be employed. Drying temperatures are also not limited, and for example, drying may be carried out at room temperature to 200°C for 10 seconds to several days. Heat treatment at a temperature of 100 to 180°C for about 10 seconds to 5 minutes may be carried out after the drying, as necessary.

In a case in which a fiber material is such that it is to be dyed, treatment with the pretreatment liquid may be carried out before dyeing or in the same bath as in dyeing, but in the case of carrying out reduction soaping, a compound with the above specified functional group (for example, a phenolic polymer compound or the like) adsorbed in the process may fall off, and therefore the treatment with the pretreatment liquid is preferably carried out after the reduction soaping after dyeing.

The treatment temperature in the dip treatment can be 60 to 130°C. The treatment time can be 5 to 60 minutes.

The step of introducing a functional group by the pretreatment liquid is preferably carried out so that the amount of compound having the above specified functional group adhered is 1.0 to 7.0 parts by weight relative to 100 parts by weight of a fiber material. Within this range, both durable water-repellency and texture can be achieved at a high level.

The pH of the pretreatment liquid may be adjusted to less than 7, for example, 3 to 5. The pH adjustment can be carried out by using a pH adjuster such as acetic acid or malic acid.

A salt can be used in combination with the pretreatment liquid to adsorb the compound having the aforementioned specific functional group effectively onto the fiber material by a salting effect. Examples of the salt that can be used include, for example, sodium chloride, sodium carbonate, ammonium sulfate, and sodium sulfate.

In the step of introducing the functional group by the pretreatment liquid, an excess amount of the compound having the aforementioned specific functional group, which has been given by the treatment, is preferably removed. Examples of the removal method include washing with water. Sufficient removal can avoid inhibition of development of water-repellency in the subsequent water-repellent treatment, and additionally, the textile product to be obtained has the favorable texture. The resulting functional group-containing fiber is preferably fully dried prior to contact with the treatment agent described above.

Examples of (ii) the fiber in which the aforementioned specific functional group has been introduced directly into the material forming the fiber include a cation-dyeable polyester (CD-PET).

In view of favorable water-repellency of the textile products to be obtained, the functional group-containing fiber preferably has a zeta potential of its surface of - 100 to -0.1 mV and more preferably -50 to -1 mV. The zeta potential of the fiber surface can be measured, for example, using a zeta potential and particle size measurement system, ELSZ-1000ZS (manufactured by Otsuka Electronics Co., Ltd.).

Embodiments have been described above, but it will be understood that various modifications can be made to embodiments and details without departing from the spirit and the scope of the claims.

### Examples

The present disclosure will be described in more detail below by way of Examples, but the present disclosure is not limited to these Examples.

### <Test Method>

The test procedures are as follows.

### [Evaluation of water-repellency (droplet fall test)]

A PET fabric (basis weight: 88 g/ m², 70 denier, gray) was immersed in a dispersion or a solution containing the fluorine-containing compound (having a concentration of the fluorine-containing compound of 0.2 g/ml), and then passed through a mangle, and heated at 70°C for 1 hour to provide a treated PET fabric. The PET fabric was fixed on a stage inclined at 30° relative to the horizontal in an automatic contact angle meter (DropMaster 701 made by Kyowa Interface Science Co., Ltd.). 20 µl of water was dropped on the PET fabric from a microsyringe, and whether the dropped water fell or not was observed. Cases where droplets fell were evaluated as "○", and cases where the droplets did not move from the position where they were dropped or were absorbed into the fabric were evaluated as "×".

### [Evaluation of oil-repellency (oil-resistance test)]

### (Preparation of treated paper)

A pulp slurry in which the weight ratio of LBKP (broad leaf tree bleached kraft pulp) was 60% by weight and the weight ratio of NBKP (needle bleached kraft pulp) was 40% by mass and in which the pulp had a freeness (Canadian Standard Freeness) of 400 ml was prepared as wood pulp. To the pulp slurry were added wet paper strength agent and a sizing agent, and paper having a paper density of 0.58 g/cm³ and a basis weight of 45 g/m² was prepared by Fourdrinier machine and was used as a base paper for external addition (size press). The base paper had an oil resistance (KIT value) of 0 and a water resistance (Cobb value) of 52 g/m².

A 1.0 wt% water dispersion or aqueous solution of the fluorine-containing compound was prepared, and an operation of applying the dispersion or solution to the base paper by a baker applicator whose gap was set to 0 mil (0 µm) or 7 mil (170 µm) and drying was repeated three times. The resultant was annealed at 100°C for 10 minutes to give a treated paper.

### (Evaluation of corn oil resistance)

Corn oil was placed on a surface of the treated paper, and when the test oil was wiped off after 15 seconds, the treated paper is evaluated for the presence or absence of oil staining.

In the case of no oil stains on the back observed, it was evaluated as "○", and in the case of oil stains on the back being observed, it was evaluated as "×".

### [Example 1]

5.0 g of polyglycerol (average molecular weight 500, 10 mmol), 20 mL of pyridine, and 7-(trifluoromethoxy)heptanoyl chloride (85 mmol) were put in a reactor, and the mixture was heated and stirred at an oil bath temperature of 60°C. The mixture was separated and purified to give a target fluorine-containing compound, which was a polyglycerol derivative.

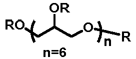

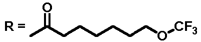

### [Example 2]

The target fluorine-containing compound, which was a polyglycerol derivative, was prepared in the same manner as in Example 1 except for changing 7-(trifluoromethoxy)heptanoyl chloride to 3-(trifluoromethoxy)propanoyl chloride.

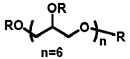

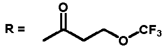

### [Example 3]

The target fluorine-containing compound, which was a polyglycerol derivative, was prepared in the same manner as in Example 1 except for changing 7-(trifluoromethoxy)heptanoyl chloride to 2-(4-(trifluoromethoxy)phenyl)acetyl chloride.

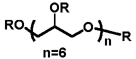

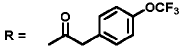

### [Example 4]

The target fluorine-containing compound, which was a polyglycerol derivative, was prepared in the same manner as in Example 1 except for changing 7-(trifluoromethoxy)heptanoyl chloride to 4-(trifluoromethoxy)benzoyl chloride.

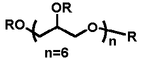

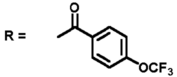

### [Example 5]

The target fluorine-containing compound, which was a polyglycerol derivative, was prepared in the same manner as in Example 1 except for changing 7-(trifluoromethoxy)heptanoyl chloride to 7-chloro-7-oxoheptyl(trifluoromethyl)carbamate.

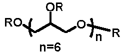

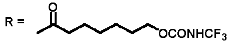

### [Example 6]

The target fluorine-containing compound, which was a polyglycerol derivative, was prepared in the same manner as in Example 1 except for changing 7-(trifluoromethoxy)heptanoyl chloride to 7-((trifluoromethyl)amino)heptanoyl chloride.

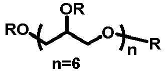

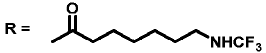

### [Example 7]

1.8 g of sorbitol (10 mmol), 20 mL of pyridine and 7-(trifluoromethoxy)heptanoyl chloride (60 mmol) were put in a reactor, and the mixture was heated and stirred at an oil bath temperature of 60°C. The mixture was separated and purified to give a target fluorine-containing compound, which was a sorbitol derivative.

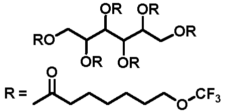

### [Example 8]

The target fluorine-containing compound, which was a sorbitol derivative, was prepared in the same manner as in Example 7 except for changing 7-(trifluoromethoxy)heptanoyl chloride to 3-(trifluoromethoxy)propanoyl chloride.

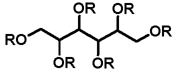

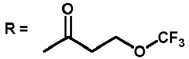

### [Example 9]

2.8 g (15 mmol) of citric acid (15 mmol) and 6-(trifluoromethoxy)hexan-1-amine (48 mmol) were put in a reactor equipped with a reflux condenser and a Dean-Stark trap, and the mixture was heated to an oil bath temperature of 70°C. Toluene was also added thereto and the mixture was heated and stirred at 135°C. The reactor was cooled to room temperature, and the mixture was separated and purified to give a target fluorine-containing compound, which was a citric acid derivative.

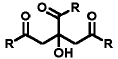

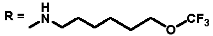

### [Example 10]

The target fluorine-containing compound, which was a citric acid derivative, was prepared in the same manner as in Example 9 except for changing 6-(trifluoromethoxy)hexan-1-amine to 2-(trifluoromethoxy)ethan-1-amine.

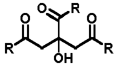

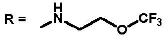

### [Example 11]

The target fluorine-containing compound, which was a citric acid derivative, was prepared in the same manner as in Example 9 except for changing 6-(trifluoromethoxy)hexan-1-amine to 4-(trifluoromethoxy)aniline.

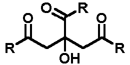

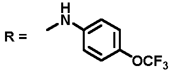

### [Example 12]

The target fluorine-containing compound, which was a citric acid derivative, was prepared in the same manner as in Example 9 except for changing 6-(trifluoromethoxy)hexan-1-amine to 6-aminohexyl(trifluoromethyl)carbamate.

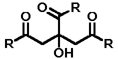

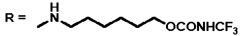

### [Example 13]

The target fluorine-containing compound, which was a citric acid derivative, was prepared in the same manner as in Example 9 except for changing 6-(trifluoromethoxy)hexan-1-amine to N-(trifluoromethyl)hexane-1,6-diamine.

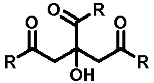

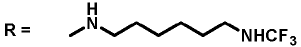

### [Example 14]

0.29 g (4.8 mmol) of ethylenediamine, 11 mmol of 7-(trifluoromethoxy)heptanoic acid, 0.12 g of 4,4-dimethylaminopyridine and chloroform were mixed, and 2.1 g of 1(3-dimethylaminopropyl)-3-ethylcarbodiimide was added thereto. Then the mixture was heated and stirred at an oil bath temperature of 50°C. The reaction solution was cooled to room temperature, and then separated and purified to give a target fluorine-containing compound, which was an ethylenediamine derivative (diamide form).

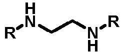

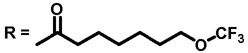

### [Example 15]

The target fluorine-containing compound, which was an ethylenediamine derivative, was prepared in the same manner as in Example 14 except for changing 7-(trifluoromethoxy)heptanoic acid to 3-(trifluoromethoxy)propanoic acid.

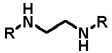

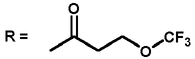

### [Example 16]

The target fluorine-containing compound, which was an ethylenediamine derivative, was prepared in the same manner as in Example 14 except for changing 7-(trifluoromethoxy)heptanoic acid to 2-(4-(trifluoromethoxy)phenyl)acetic acid.

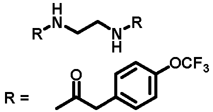

### [Example 17]

The target fluorine-containing compound, which was an ethylenediamine derivative, was prepared in the same manner as in Example 14 except for changing 7-(trifluoromethoxy)heptanoic acid to 4-(trifluoromethoxy)benzoic acid. This compound was subjected to the droplet fall test and the oil-resistance test, and was determined as "○".

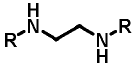

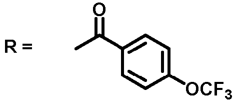

### [Example 18]

The target fluorine-containing compound, which was an ethylenediamine derivative, was prepared in the same manner as in Example 14 except for changing 7-(trifluoromethoxy)heptanoic acid to 7-(((trifluoromethyl)carbamoyl)oxy)heptanoic acid.

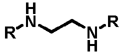

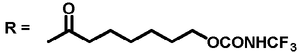

### [Example 19]

The target fluorine-containing compound, which was an ethylenediamine derivative, was prepared in the same manner as in Example 14 except for changing 7-(trifluoromethoxy)heptanoic acid to 7-((trifluoromethyl)amino)heptanoic acid.

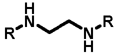

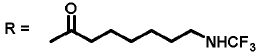

### [Example 20]

4-(trifluoromethoxy)phenylacetic acid (Tokyo Chemical Industry Co., Ltd.), glycerol and 4,4-dimethylaminopyridine were dissolved in dehydrated toluene, and then 1-(3-dimethylaminopropyl)-3-ethylcarbodiimide was added thereto, and the mixture was stirred overnight at an oil bath temperature of 50°C.

The reaction solution was cooled to room temperature and then concentrated to give a solid, and the solid was washed with methanol and diethyl ether, and dried to give a fluorine-containing compound, which was a modified body of polyol prepared by modifying glycerol with 4-(trifluoromethoxy)phenylacetic acid (Tokyo Chemical Industry Co., Ltd.).

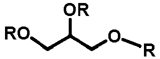

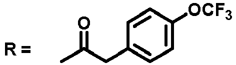

### [Example 21]

4-(trifluoromethoxy)benzoic acid (Tokyo Chemical Industry Co., Ltd.), glycerol and 4, 4-dimethylaminopyridine were dissolved in dehydrated toluene, and then 1-(3-dimethylaminopropyl)-3-ethylcarbodiimide was added thereto, and the mixture was stirred overnight at an oil bath temperature of 50°C.

The reaction solution was cooled to room temperature and then concentrated to give a solid, and the solid was washed with methanol and diethyl ether, and dried to give a fluorine-containing compound, which was a modified body of polyol prepared by modifying glycerol with 4-(trifluoromethoxy)benzoic acid.

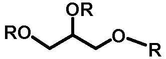

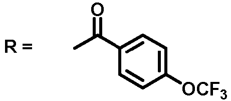

### [Example 22]

Glycerol, dehydrated toluene and 4-(trifluoromethoxy)phenyl isocyanate (Tokyo Chemical Industry Co., Ltd.) were put in a reactor and a drop of dibutyltin dilaurate was added thereto, and the mixture was stirred at 60°C for 1 hour.

After 4-(trifluoromethoxy)phenyl isocyanate (Tokyo Chemical Industry Co., Ltd.) was found to disappear, the mixture was added dropwise to hexane to precipitate solid. The precipitated solid was recovered by suction filtration to give a fluorine-containing compound, which was a modified body of polyol prepared by modifying glycerol with 4-(trifluoromethoxy)phenyl isocyanate.

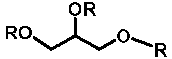

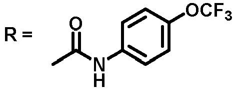

### [Example 23]

Glycerol, a stirring bar, ethylene diamine, toluene and 4-(trifluoromethoxy)phenyl isocyanate were put in a reactor and a drop of dibutyltin dilaurate was added thereto, and the mixture was stirred at 60°C for 1 hour.

After 4-(trifluoromethoxy)phenyl isocyanate was found to disappear, the mixture was added dropwise to hexane to precipitate solid. The precipitated solid was recovered by suction filtration to give a fluorine-containing compound, which was a modified body of polyvalent amine prepared by modifying ethylene diamine with 4-(trifluoromethoxy)phenyl isocyanate (Tokyo Chemical Industry Co., Ltd.).

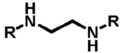

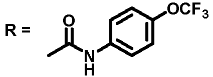

### [Example 24]

4-(trifluoromethoxy)phenol, citric acid and 4, 4-dimethylaminopyridine were dissolved in toluene, and then 1-(3-dimethylaminopropyl)-3-ethylcarbodiimide was added thereto, and the mixture was stirred overnight at an oil bath temperature of 50°C.

The reaction solution was cooled to room temperature and then concentrated to give a solid, and the solid was washed with methanol and diethyl ether, and dried to give a fluorine-containing compound, which was a modified body of polyvalent carboxylic acid prepared by modifying citric acid with 4-(trifluoromethoxy)phenol.

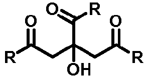

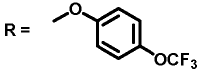

### [Example 25]

5.0 g of monoglycerol, 20 mL of pyridine and 4-(trifluoromethoxy)benzoyl chloride (85 mmol) were put in a reactor, and the mixture was heated and stirred at an oil bath temperature of 60°C. The mixture was separated and purified to give a target fluorine-containing compound, which was a monoglycerol derivative. This compound was subjected to the droplet fall test and the oil-resistance test and was determined as "○".

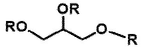

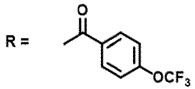

The fluorine-containing compound of the present disclosure imparts excellent liquid-repellency to a substrate.

## Claims

1. A fluorine-containing compound comprising:
a moiety derived from a compound (a) having one or more active hydrogen-containing groups selected from a hydroxy group, an amino group, a carboxyl group and a thiol group; and
a moiety derived from a compound (b) having R^{f1} or R^{f2}, wherein
R^{f1} is -CF₃, -CF₂H or -CFH₂,
R^{f2} is -CF₂- or -CFH-, and
R^{f1} and R^{f2} do not constitute a moiety of a fluoroalkyl group having 2 or more carbon atoms.

2. The fluorine-containing compound according to claim 1, wherein the fluorine-containing compound has an oxygen atom or a nitrogen atom in a position adjacent to the R^{f1}, and has an oxygen atom or a nitrogen atom in at least one of positions adjacent to the R^{f2}.

3. The fluorine-containing compound according to claim 1 or 2, wherein
the R^{f1} is CF₃-, and
the R^{f2} is -CF₂-.

4. The fluorine-containing compound according to any one of claims 1 to 3, wherein the compound (b) has a group represented by the formula:
-Y^{f}-Z^{f}_{α}
wherein, independently at each occurrence,
Y^{f} is a 1+α valent group composed of one or more selected from the group consisting of Y^{f1} and Y^{f2},
Y^{f1} is a group composed of one or more selected from a direct bond, -O-, -C(=O)-, -C(=NR')-, -S-, -S(=O)₂-, - NR'-, -C(OR')R'-, -C(OR')(-)₂ and -N(-)₂, wherein R' is a hydrogen atom or an organic group,
Y^{f2} is a group composed of one or more selected from the group consisting of an aliphatic group and an aromatic group,
Z^{f} is R^{f1}, or a hydrocarbon group having 2 or more and 40 or less carbon atoms and including R^{f1} or R^{f2}, and
α is 1 to 3.

5. The fluorine-containing compound according to any one of claims 1 to 4, wherein the compound (b) has a group represented by the formula:
-Y^{f11}-(Y^{f21}-Y^{f12})_{β}-Y^{f22}-Y^{f13}-Z^{f}
wherein, independently at each occurrence,
Y^{f11} is a direct bond, -O-, -NH-, -C(=O)-, -C(=O)-NH- or -S-,
Y^{f21} is a group composed of one or more selected from the group consisting of an aliphatic group and an aromatic group,
Y^{f12} is a group composed of one or more selected from -O-, -C(=O)-, -C(=NR')-, -S-, -S(=O)₂-, -NR'- and - C(OR')R'-, wherein R' is a hydrogen atom or an organic group,
β is 0 to 3,
Y^{f22} is a group composed of one or more selected from the group consisting of an aliphatic group and an aromatic group,
Y^{f13} is -O-, -O-C(=O)-O-, -O-C(=O)-NR'-, -NR'-, -NR'-C(=O)-O-, -NR'-C(=O)-NR'-, -C(=O)-O-, -C(=O)-NR'- or - SO₂NR'-, wherein R' is a hydrogen atom or an organic group, and
Z^{f} is R^{f1}, or a hydrocarbon group having 2 or more and 40 or less carbon atoms and including R^{f1} or R^{f2}.

6. The fluorine-containing compound according to claim 5, wherein
Y^{f22} is a group represented by the formula:
-Y^{f221}-Y^{f222}-
wherein
Y^{f221} is a direct bond or a hydrocarbon group having 2 to 40 carbon atoms, and
Y^{f222} is a direct bond or a phenyl group,
Y^{f13} is -O- or -NR'-, wherein R' is a hydrogen atom or an organic group,
β is 0 or 1, and
Z^{f} is R^{f1}.

7. The fluorine-containing compound according to any one of claims 1 to 6, wherein the compound (b) is an active hydrogen-reactive compound; a polymerizable monomer or a polymer thereof.

8. The fluorine-containing compound according to any one of claims 1 to 7, wherein the compound (a) is a natural product.

9. The fluorine-containing compound according to any one of claims 1 to 8, wherein the compound (a) is polyol, polyamine or polycarboxylic acid.

10. The fluorine-containing compound according to any one of claims 1 to 9, wherein the compound (a) is a monosaccharide, an oligosaccharide, a polysaccharide, a sugar alcohol, a hydroxy acid, an amino acid, vitamin, flavonol, hydroxyhydrocarbon, a polymer of a hydroxy group-containing compound, an organic acid, an organic amine, or a saturated/unsaturated aliphatic hydrocarbon compound.

11. The fluorine-containing compound according to any one of claims 1 to 10, wherein the compound (a) is
glucose, fructose, galactose, xylose;
sucrose, cycloamylose, cyclodextrin, maltose, trehalose, lactose, sucralose;
sorbitol, maltitol, erythritol, isomalt, lactitol, mannitol, xylitol, sorbitan, lactitol;
starch, cellulose, curdlan, pullulan, alginic acid, carrageenan, guar gum, chitin, chitosan, locust bean gum, kappa-carrageenan, iota-carrageenan, isomaltodextrin, gellan gum, tamarind seed gum;
ascorbic acid, kojic acid, quinic acid, chlorogenic acid, gluconic acid;
glucosamine;
inositol;
catechin, quercetin, anthocyanin;
glycerol, ethylene glycol, propylene glycol, diethylene glycol, triethylene glycol, tetraethylene glycol, neopentyl glycol, trimethylene glycol, trimethylolpropane, trimethylolethane;
polyglycerol, polyvinyl alcohol, hydroxyethyl (meth)acrylate polymer, hydroxypropyl (meth)acrylate polymer and hydroxybutyl(meth)acrylate polymer;
citric acid, malic acid, glutaric acid, adipic acid, phthalic acid, alginic acid, tartaric acid, oxalic acid, malonic acid, succinic acid, fumaric acid, maleic acid, aldaric acid;
tricarballylic acid, t-aconitic acid, trimellitic acid;
pyromellitic acid, itaconic acid;
alkylene diamine, alkylene triamine, aromatic diamine; or
a saturated/unsaturated aliphatic hydrocarbon compound having an active hydrogen group.

12. The fluorine-containing compound according to any one of claims 1 to 11, wherein the fluorine-containing compound has a biobased content of 20% or more.

13. The fluorine-containing compound according to any one of claims 1 to 12, wherein the fluorine-containing compound has a weight average molecular weight of 1,000 or more.

14. The fluorine-containing compound according to any one of claims 1 to 13, wherein the fluorine-containing compound has a weight average molecular weight of less than 1,000.

15. The fluorine-containing compound according to any one of claims 1 to 14, wherein
the fluorine-containing compound has a monovalent hydrocarbon group having 2 or more and 40 or less carbon atoms as a modifying group other than -Y^{f}-Z^{f}ₙ.

16. The fluorine-containing compound according to claim 11, wherein the compound (b) has a group represented by the formula:
-Y^{f11}-(Y^{f21}-Y^{f12})_{β}-Y^{f22}-Y^{f13}-Z^{f}
wherein, independently at each occurrence,
Y^{f11} is a direct bond, -O-, -NH-, -C(=O)-, -C(=O)-NH- or -S-,
Y^{f21} is a group composed of one or more selected from the group consisting of an aliphatic group and an aromatic group,
Y^{f12} is a group composed of one or more selected from -O-, -C(=O)-, -C(=NR')-, -S-, -S(=O)₂-, -NR'- and - C(OR')R'-, wherein R' is a hydrogen atom or an organic group,
β is 0 to 3,
Y^{f22} is a group composed of one or more selected from the group consisting of an aliphatic group and an aromatic group,
Y^{f13} is -O-, -O-C(=O)-O-, -O-C(=O)-NR'-, -NR'-, -NR'-C(=O)-O-, -NR'-C(=O)-NR'-, -C(=O)-O-, -C(=O)-NR'- or - SO₂NR'-, wherein R' is a hydrogen atom or an organic group, and
Z^{f} is -CF₃.

17. A dispersion comprising the fluorine-containing compound according to any one of claims 1 to 16 and a liquid medium.

18. A water- and oil-repellent agent comprising the fluorine-containing compound according to any one of claims 1 to 16.

19. The water- and oil-repellent agent according to claim 18, which is for a textile product or a paper product.

20. A method for producing a treated substrate, comprising treating a substrate with the water- and oil-repellent agent according to claim 18 or 19.

21. The method according to claim 20, wherein the substrate is a textile product or a paper product.

22. A product to which the fluorine-containing compound according to any one of claims 1 to 16 is adhered.
